# EUROPÄISCHE PATENTANMELDUNG

(11) **EP 2 400 411 A1**
(43) Veröffentlichungstag der Anmeldung: **28.12.2011**
(21) Anmeldenummer: 10006562.2
(22) Anmeldetag: 24.06.2010
(51) Int. Cl.: G06F 19/00

(54) **Analysesystem mit erweiterter Benutzerinformation**

(71) Anmelder: Roche Diagnostics GmbH, 68305 Mannheim (DE); F.Hoffmann-La Roche AG, 4070 Basel (CH)
(72) Erfinder: Die Erfindernennung liegt noch nicht vor
(74) Vertreter: Silber, Anton

(57) **Zusammenfassung**

Die vorliegende Erfindung betrifft ein Analysesystem mit erweiterter Benutzerinformation, umfassend
- ein tragbares analytisches Messgerät zur Messung von Stoffkonzentrationen in fluiden Medien, das eine erste Datenempfangsvorrichtung zum Auslesen von in einem beschreibbaren nichtflüchtigen Datenspeicher gespeicherten Daten und ein Anzeigeelement zum Anzeigen von Messergebnissen aufweist;
- eine Verpackungseinheit für Verbrauchsmittel, die zur Messung von Stoffkonzentrationen in fluiden Medien und/oder zur Vorbereitung einer solchen Messung verwendet werden, wobei der beschreibbare nichtflüchtige Datenspeicher der Verpackungseinheit zugeordnet ist;
- eine erste Datenübertragungsvorrichtung zum Übertragen von Steuerungsdaten auf den beschreibbaren nichtflüchtigen Datenspeicher; und
- eine zweite Datenübertragungsvorrichtung zum Übertragen von Anzeigedaten auf den beschreibbaren nichtflüchtigen Datenspeicher;

wobei das Anzeigeelement des tragbaren analytischen Messgerätes zum Anzeigen mindestens eines Teils der Anzeigedaten eingerichtet ist.
Weiterhin betrifft die vorliegende Erfindung ein Analyseverfahren mit erweiterter Benutzerinformation, bei welchem z.B. ein erfindungsgemäßes Analysesystem zum Einsatz kommen kann.

## Beschreibung

Die vorliegende Erfindung betrifft ein Analysesystem, umfassend ein tragbares analytisches Messgerät zur Messung von Stoffkonzentrationen in fluiden Medien mit einem Anzeigeelement zum Anzeigen von Messergebnissen, wobei das Messgerät zur Durchführung von Analysen Daten (z. B. Steuerungsdaten) und Verbrauchsmaterialien verwendet. Weiterhin betrifft die vorliegende Erfindung ein Analyseverfahren, das mit einem solchen Analysesystem durchgeführt werden kann.

Analytische Messgeräte, welche Stoffkonzentrationen in fluiden Medien messen und so dimensioniert sind, dass sie von einem Anwender bequem beziehungsweise unauffällig mitgeführt werden können, sind allgemein bekannt. Solche Messgeräte werden beispielsweise zur Messung von Stoffkonzentrationen in Blut verwendet, insbesondere zur Bestimmung von Blutglukose-oder Blutfettkonzentrationen sowie zur Bestimmung von Koagulationswerten des Blutes. Üblicherweise werden hierbei die zu analysierenden Blutproben auf Verbrauchsmaterialien wie Teststreifen aufgegeben. Die in einem Analysebereich des Verbrauchsmaterials infolge einer Reaktion zwischen dem zu bestimmenden Analyten und einer oder mehrerer Reagenz-Komponenten des Verbrauchsmaterials stattfindende Veränderung wird dann durch das analytische Messgerät ausgewertet. Die unter Verwendung von Daten zur Kalibrierung von dem Messgerät aus den Messdaten ermittelten Messergebnisse werden in der Regel auf einem Anzeigeelement des Messgerätes dargestellt. Auch kontinuierlich messende Systeme, die z.B. mit in die Haut insertierbaren Glukosesensoren arbeiten, sind bekannt und finden bereits Anwendung.

Zur Übertragung von Steuerungsdaten, beispielsweise chargenspezifischer Kalibrierungsdaten, auf das tragbare analytische Messgerät werden unterschiedliche Übertragungstechniken und Datenspeicher eingesetzt. Hierbei kommt es insbesondere darauf an sicherzustellen, dass eine Übertragung solcher Steuerungsdaten tatsächlich erfolgt und dass diese Übertragung fehlerfrei erfolgt, damit das Messgerät seine analytischen Funktionen korrekt ausführt.

Die US 6,176,119 beschreibt ein Analysensystem für Probenflüssigkeiten, bei welchem dem Analysesystem Testelemente mittels eines Testelementevorratsbehältnisses zugeführt werden, wobei das Testelementevorratsbehältnis mit Steuerungsdaten in Form einer chargenspezifischen Codierung versehen ist. Die Codierung kann dabei insbesondere in optischen oder elektromagnetischen Codes wie Strichcodes, Magnetstreifencodes oder rein elektronischen Speichern wie z.B. Read-Only-Memory (ROM)-Bausteinen bestehen.

Bei dem in der WO 2006/069675 A1 beschriebenen tragbaren analytischen Messsystem werden chargenspezifische Daten drahtlos von einem Teststreifenbehältnis auf eine Datenempfangsvorrichtung des analytischen Messsystems übertragen, beispielsweise mittels eines Transponders. Das Messsystem ist in der Lage, die Anzahl/Zeitdauer zu erfassen, wie oft/wie lange das Teststreifenbehältnis geöffnet wird/ist.

Aus der US 5,366,609 ist ein Biosensor-Messgerät mit einem steckbaren Speicherbaustein bekannt, der in eine elektrische Kupplung des Messgerätes eingesetzt werden kann und auf diese Weise Daten zur Steuerung von Funktionen des Messgerätes auf das Messgerät übertragen kann.

Aus der WO 2006/026748 A1 ist ein Verfahren zur Kalibrierung eines analytischen Sensors bekannt, bei dem zumindest ein Teil der Kalibrationsinformation drahtlos auf den Sensor übertragen werden kann, insbesondere während oder nach der Produktion sowie im verpackten Zustand des Sensors.

Die US 2006/0,182,656 A1 beschreibt unter anderem einen Dispenser für Teststreifen, der via RFID-tag Teststreifen-bezogene Informationen an ein separates Analysegerät übertragen kann.

Die WO 2006/040083 A1 beschreibt ein Analysensystem zur Konzentrationsbestimmung von Körperflüssigkeiten, das ein analytisches Testelement und ein davon separates Instrument mit einer Einheit zur Datenauswertung enthält, wobei das Testelement elektrische Komponenten aufweist, die mindestens teilweise auf der Basis von Polymerelektronik ausgebildet sind. Hierbei können von einem Sendemodul des Testelementes Daten zu einem Lesemodul des Instrumentes übermittelt werden.

Die vorgenannten Veröffentlichungen über Messsysteme betreffen alle die sichere und fehlerfreie Übertragung von Daten zur Steuerung analytischer Funktionen der Messsysteme von einem Datenspeicher auf ein Messgerät. Diese Steuerungsdaten werden stets bei der werksseitigen Herstellung bzw. Verpackung der für die Messsysteme vorgesehenen Verbrauchsmaterialien auf den jeweiligen, dem Verbrauchsmaterial beigefügten Datenspeicher übertragen. Eine spätere Ergänzung bzw. Aktualisierung der einmal gespeicherten Daten ist nicht vorgesehen und in der Regel nicht ohne vertretbaren Aufwand möglich. Dies wäre jedoch insbesondere in Fällen wünschenswert, in denen aktualisierte Steuerungsdaten Reparaturdaten zur Korrektur fehlerhafter Funktionen, wie zum Beispiel Softwarefunktionen, des analytischen Messgerätes oder Upgradedaten zur Aktualisierung der Funktionen des analytischen Messgerätes umfassen könnten.

Zudem bedeutet die Verwendung der vorgestellten tragbaren analytischen Messgeräte für den Anwender stets einen gewissen Aufwand, verschiedene Informationen zur korrekten Handhabung aktuell und im Anwendungszeitpunkt verfügbar bereitzuhalten. So müssen in der Regel einerseits die zur Messung benötigten Verbrauchsmaterialien korrekt mit dem jeweiligen Messgerät zusammengeführt werden, andererseits können vielfältige weitere Informationen in diesem Zusammenhang vom Anwender vorteilhaft genutzt oder benötigt werden. Dies können allgemeine und spezielle Anwendungshinweise sein, wie zum Beispiel Zeit, Dauer und Häufigkeit der Anwendung sowie Dosierungsempfehlungen. Derartige Daten erhält der Anwender in der Regel von einem Arzt, Apotheker oder einer Krankenschwester, häufig in mündlicher oder handschriftlicher Form. Es wäre jedoch wünschenswert, derartige anwendungsrelevante Daten mittels des beim Anwender ohnehin vorhandenen tragbaren analytischen Messgerätes zur Verfügung zu stellen. In diesem Zusammenhang ist auch das Bereitstellen weiterer allgemeiner Daten mittels des Messgerätes von Interesse, beispielsweise von Kontaktdaten und Öffnungszeiten von Ärzten, Arztpraxen und Apotheken. Darüber hinaus wäre es wünschenswert, die Darstellung der zuvor genannten Daten (aber auch z. B. von Messergebnissen) auf einem Display des Messgerätes an individuelle Bedürfnisse eines Anwenders anzupassen, ohne dass hierfür zusätzliche Bedienhandlungen seitens des Anwenders erforderlich sind. So könnten dieselben Daten quasi automatisch beispielsweise mit unterschiedlicher Schriftgröße, Zeichendichte, Zeilenabstand, Menge der zeitgleich angezeigten Informationen, etc. wunschgemäß oder auch altersgruppengerecht angezeigt werden.

Es war daher Aufgabe der vorliegenden Erfindung, ein Analysesystem zur Verfügung zu stellen, welches zusätzlich zur Anzeige von Messergebnissen auch die Anzeige aktueller weiterer Daten mittels eines tragbaren analytischen Messgerätes ermöglicht. Solche aktuellen weiteren Daten sollten auf einfache Weise insbesondere von einer Stelle in das Analysesystem eingespeist werden können, die mit Vertrieb, Distribution, Verteilung und/oder Ausgabe von Verbrauchsmaterial für ein derartiges Messgerät befasst ist.

Eine weitere Aufgabe der vorliegenden Erfindung war es, ein Analysesystem zur Verfügung zu stellen, welches zumindest teilweise eine Rückübertragung von Daten, die beim Anwender eines tragbaren analytischen Messgerätes ermittelt wurden, an eine dem Anwender vorgeschaltete Stelle des Analysesystems ermöglicht. Eine vorgeschaltete Stelle sollte hierbei insbesondere eine solche sein, die mit Vertrieb, Distribution, Verteilung, Ausgabe und/oder Herstellung von Verbrauchsmaterial für ein derartiges Messgerät befasst ist, und/oder die mit Herstellung derartiger Messgeräte befasst ist.

Diese Aufgaben werden gelöst durch ein Analysesystem mit erweiterter Benutzerinformation gemäß den Ansprüchen 1 und 10, sowie durch ein Analyseverfahren gemäß Anspruch 11.

Bevorzugte Ausführungsformen der Erfindung sind in den Unteransprüchen beschrieben.

Ein erster Gegenstand der Erfindung betrifft ein Analysesystem mit erweiterter Benutzerinformation, umfassend
- ein tragbares analytisches Messgerät zur Messung von Stoffkonzentrationen in fluiden Medien, das eine erste Datenempfangsvorrichtung zum Auslesen von in einem beschreibbaren nichtflüchtigen Datenspeicher gespeicherten Daten und ein Anzeigeelement zum Anzeigen von Messergebnissen aufweist;
- eine Verpackungseinheit für Verbrauchsmittel, die zur Messung von Stoffkonzentrationen in fluiden Medien und/oder zur Vorbereitung einer solchen Messung verwendet werden, wobei der beschreibbare nichtflüchtige Datenspeicher der Verpackungseinheit zugeordnet ist;
- eine erste Datenübertragungsvorrichtung zum Übertragen von Steuerungsdaten auf den beschreibbaren nichtflüchtigen Datenspeicher; und
- eine zweite Datenübertragungsvorrichtung zum Übertragen von Anzeigedaten auf den beschreibbaren nichtflüchtigen Datenspeicher;
wobei das Anzeigeelement des tragbaren analytischen Messgerätes zum Anzeigen mindestens eines Teils der Anzeigedaten eingerichtet ist.

Das tragbare analytische Messgerät misst üblicherweise eine Analytkonzentration, zum Beispiel Glukose oder Fette wie Triglyceride, insbesondere Glukose, in einer Körperflüssigkeit, insbesondere Blut oder interstitielle Flüssigkeit. Das Messgerät ist vorzugsweise so dimensioniert, dass es bequem vom Anwender in einer Hand gehalten werden kann. Es besitzt vorzugsweise im Wesentlichen die Form eines flachen, länglichen Quaders, dessen Kanten und/oder Ecken abgerundet sein können. Bei dieser Bauform können in der Regel eine Vorder- und Rückseite und seitliche Flächen des Gerätegehäuses unterschieden werden. Die obere Seitenfläche wird hierin auch als Stirnseite bezeichnet, die untere Seitenfläche als Unterseite. Das Gehäuse des Messgerätes ist aus einem leichten und stabilen Werkstoff, vorzugsweise einem Leichtmetall, einem Kunststoff oder einer Kombination davon, gefertigt. Auf der Außenseite des Gerätegehäuses können sich je nach gewünschtem Bedienkomfort erforderliche Schalter befinden, beispielsweise Schalter zum Ein-und Ausschalten des Gerätes sowie ggf. Bedienungsknöpfe für weitere Funktionen, wie Uhrzeitanzeige, Speicherung der Messwerte etc. In der Regel umfasst das tragbare analytische Messgerät weitere Komponenten, wie zum Beispiel eine elektrische Kupplung zur Herstellung eines elektrischen Kontaktes mit anderen Objekten, insbesondere dem beschreibbaren Datenspeicher; eine Kontrolleinheit, insbesondere zur Steuerung der Mess- und/oder Anzeigefunktionen, zur Steuerung des Datenflusses und/oder der Datenspeicherung; Komponenten zur Durchführung elektrochemischer oder photometrischer Messungen zur Bestimmung von Stoffkonzentrationen in fluiden Medien; sowie Mikroprozessoren, Speicherbausteine, etc. Die weiteren Komponenten, insbesondere die vorstehend beispielhaft aufgezählten, sind dem Fachmann hinlänglich bekannt und können von ihm ohne Weiteres für die Zwecke der vorliegenden Erfindung eingesetzt oder gegebenenfalls modifiziert werden.

Speziell im Fall kontinuierlich messender Systeme kann das tragbare analytische Messgerät zwei- oder mehrteilig ausgebildet sein. Dies kann insbesondere realisiert werden, indem die Probenahme mittels eines in die Haut insertierbaren (Glukose-)Sensors, der in der Regel zum Einmalgebrauch vorgesehen ist, vorgenommen wird. Der insertierbare Sensor wird üblicherweise zusammen mit einem mehrfach verwendbaren und am Körper fixierbaren ersten Geräteteil eingesetzt, wobei dieses erste Geräteteil Messdaten bzw. Messergebnisse an ein zweites, von dem ersten Geräteteil getrenntes Geräteteil übermitteln kann. Diese Übermittlung kann via Kabelverbindung oder vorzugsweise drahtlos erfolgen. Hierbei enthält das zweite Geräteteil vorteilhafterweise das Anzeigeelement.

Weiterhin weist das Gerätegehäuse des tragbaren analytischen Messgerätes eine Öffnung zur Aufnahme der Verbrauchsmittel, die zur Messung der Stoffkonzentration verwendet werden, z. B. ein einzelner Teststreifen, auf. Es können Teststreifen eingesetzt werden, die einen Transponder, insebondere einen RFID-tag (RFID bedeutet Radio frequency identification), aufweisen, siehe z.B. WO 2006/040083. Hierbei kann das Verbrauchsmittel vollständig oder teilweise durch die Öffnung in das Gerätegehäuse eingeführt werden. Üblicherweise wird man das einzelne Verbrauchsmittel nur teilweise in das Gerätegehäuse einführen. In diesem Fall ist eine Applikation des zu analysierenden fluiden Mediums auf dem Teil des Verbrauchsmittels, der aus dem Gerätegehäuse heraus ragt, auf einfache Weise möglich. Wenn die Applikation des fluiden Mediums auf dem Verbrauchsmittel bereits vor dem Einführen in das Gerätegehäuse erfolgt, so kann das Verbrauchsmittel vollständig in das Gehäuse eingeführt werden.

Alternativ kann das Gerätegehäuse des tragbaren analytischen Messgerätes ein Aufnahmeelement zur Aufnahme der Verpackungseinheit der Verbrauchsmittel aufweisen. Dies ist insbesondere zweckmäßig, wenn die Verpackungseinheit ein Behältnis zur magazinierten Aufbewahrung mehrerer der Verbrauchsmittel, die zur Messung der Stoffkonzentration verwendet werden, ist (z. B. Teststreifen-Magazine). Das Aufnahmeelement zur Aufnahme der Verpackungseinheit ist im einfachsten Fall als Öffnung im Gerätegehäuse ausgestaltet, in welche die Verpackungseinheit (z.B. ein Verbrauchsmittel-Magazin) teilweise oder vollständig eingeführt werden kann.

Werden die Verbrauchsmittel, die zur Messung der Stoffkonzentration verwendet werden, z. B. ein einzelner Teststreifen oder ein Teststreifen-Magazin, nur teilweise in das Gehäuse des Messgerätes eingeführt, so wird das Verbrauchsmittel oder Verbrauchsmittel-Magazin zweckmäßigerweise in, an oder auf der Öffnung bzw. dem Aufnahmeelement fixiert, z. B. eingeklemmt, eingerastet oder eingehakt. Werden die Verbrauchsmittel, die zur Messung der Stoffkonzentration verwendet werden, z. B. ein einzelner Teststreifen oder ein Teststreifen-Magazin, vollständig in das Gehäuse des Messgerätes eingeführt, so kann die Öffnung zur Aufnahme der Verbrauchsmittel bzw. das Aufnahmeelement zur Aufnahme der Verpackungseinheit mit einem Verschluss, z.B. einer Klappe, einem Deckel, einem Schieber, einer Folie oder einer sonstigen Abdeckung, zum Verschließen der Öffnung bzw. des Aufnahmeelements versehen werden.

Verbrauchsmittel, die nicht zur direkten Messung der Stoffkonzentration sondern zur Vorbereitung einer solchen Messung verwendet werden, z. B. Lanzetten zum Hervorbringen von Körperflüssigkeiten, müssen als solche nicht notwendigerweise in das analytische Messgerät eingeführt werden. Vielmehr werden solche Verbrauchsmittel üblicherweise separat von dem analytischen Messgerät verwendet. Dennoch kann auch in diesem Fall ein Aufnahmeelement, z.B. eine Öffnung im Gerätegehäuse, zur teilweisen oder vollständigen Aufnahme einer Verpackungseinheit solcher Verbrauchsmittel (z.B. eines Verbrauchsmittel-Magazins), wie zuvor beschrieben, vorgesehen werden. Zweckmäßiger ist es in diesem Fall jedoch, wenn das tragbare analytische Messgerät eine Positionierungshilfe, wie nachfolgend beschrieben, aufweist.

Das Gehäuse des tragbaren analytischen Messgerätes kann weiterhin eine Positionierungshilfe zur Positionierung der Verpackungseinheit (z.B. eines Verbrauchsmittel-Magazins) relativ zum Messgerät aufweisen. Die Positionierungshilfe kann eine (optische, insbesondere farbliche) Markierung auf der Oberfläche des Gehäuses des analytischen Messgerätes sein. Alternativ oder zusätzlich kann die Positionierungshilfe auch dreidimensional konstruktiv ausgestaltet sein. In diesem Fall ist ihre Form, beispielsweise eine Aussparung auf der Oberfläche des Messgerätegehäuses, an die Form der zu positionierenden Verpackungseinheit angepasst. Eine solche Markierung und/oder Aussparung kann beispielsweise auf der Vorder-, Rück-, Stirnseite oder einer seitlichen Fläche des Gehäuses des Messgerätes vorgesehen sein. Insbesondere kann die Positionierungshilfe derart dreidimensional ausgestaltet sein, dass eine relativ flache Vertiefung vorgesehen wird, beispielsweise an der Vorder- oder Stirnseite des Gehäuses des Messgerätes. Diese Vertiefung kann insbesondere eine Aussparung mit einer Tiefe beispielsweise im Bereich von 0,2 bis 5 mm, insbesondere im Bereich von 0,5 bis 2,5 mm sein. Eine solche flache Vertiefung kann auch dadurch realisiert werden, dass ein relativ niedriger, umlaufender oder unterbrochener Rundsteg, beispielsweise mit einer Höhe im Bereich von 0,2 bis 5 mm, insbesondere im Bereich von 0,5 bis 2,5 mm, auf der Oberfläche des Gehäuses des Messgerätes vorgesehen wird. Eine derartige flache Vertiefung bzw. ein derartig niedriger Rundsteg ermöglicht ein einfaches Ablegen bzw. Abstellen der Verpackungseinheit. Ferner kann die Positionierungshilfe so ausgestaltet sein, dass die Verpackungseinheit in, an oder auf der Positionierungshilfe fixiert, z. B. eingeklemmt, eingerastet oder eingehakt, wird. In einer bevorzugten Ausführungsform ist die Positionierungshilfe dreidimensional ausgestaltet, wie zuvor beschrieben, und zusätzlich durch eine Markierung, beispielsweise eine farbige, optische Markierung, ausgestaltet.

Die Positionierungshilfe kann zusätzlich zu der zuvor beschriebenen Öffnung zur Aufnahme von Verbrauchsmitteln bzw. zusätzlich zu dem zuvor beschriebenen Aufnahmeelement zur Aufnahme der Verpackungseinheit vorgesehen werden. Die Positionierungshilfe (z.B. Markierung und/oder Aussparung) dient dazu, die Verpackungseinheit mit dem ihr zugeordneten beschreibbaren, nichtflüchtigen Datenspeicher in definierter Weise in räumliche Nähe zu dem tragbaren analytischen Messgerät zu bringen. Sofern andererseits die Verpackungseinheit mit dem ihr zugeordneten beschreibbaren, nichtflüchtigen Datenspeicher einem Aufnahmeelement zur Aufnahme der Verpackungseinheit zugeführt wird, resultiert eine solche definierte räumliche Nähe bereits durch das Einführen der Verpackungseinheit in das Aufnahmeelement. Der Aspekt der definierten räumlichen Nähe spielt eine Rolle für die Übertragung von Daten (insbesondere drahtlos gemäß RFID-Prinzip) zwischen dem beschreibbaren, nichtflüchtigen Datenspeicher und der ersten Datenempfangsvorrichtung des tragbaren analytischen Messgerätes, wie weiter unten näher erläutert wird.

Das tragbare analytische Messgerät kann weiterhin ein eigenes Aufnahmeelement zur Aufnahme des beschreibbaren, nichtflüchtigen Datenspeichers aufweisen. Dies ist zweckmäßig, wenn der beschreibbare, nichtflüchtige Datenspeicher der Verpackungseinheit entnehmbar beigefügt ist. Ein solches Aufnahmeelement, z.B. in Form einer Öffnung oder Aussparung im Gerätegehäuse, kann insbesondere eine elektrische Kupplung umfassen, mittels derer der beschreibbare, nichtflüchtige Datenspeicher über eine Drahtverbindung ausgelesen und/oder beschrieben werden kann. Das Aufnahmeelement ist zweckmäßig an die Form des Datenspeichers angepasst, z.B. kann eine Aussparung an der Oberfläche, insbesondere an einer Seitenfläche, des Gehäuses des Messgerätes vorgesehen sein. Besonders geeignet als beschreibbarer, nichtflüchtiger Datenspeicher ist in diesem Fall ein Read-Only-Memory (ROM)-Baustein, wie er beispielsweise aus der EP 0 746 762 B1 bekannt ist, auf deren Offenbarung hiermit in vollem Umfang Bezug genommen wird, in Kombination mit einem Transponder, insbesondere einem RFID-tag.

Alternativ ist es möglich, dass kein eigenes Aufnahmeelement zur Aufnahme des beschreibbaren, nichtflüchtigen Datenspeichers vorgesehen wird, z.B. weil der Datenspeicher fest mit der Verpackungseinheit verbunden angeordnet ist. In diesem Fall weist das tragbare analytische Messgerät vorzugsweise ein Aufnahmeelement zur Aufnahme der Verpackungseinheit und/oder eine Positionierungshilfe zur Positionierung der Verpackungseinheit relativ zum analytischen Messgerät auf, wie zuvor beschrieben.

Die Spannungsversorgung des tragbaren analytischen Messgerätes kann stromnetzabhängig oder stromnetzunabhängig erfolgen; aus praktischen Gründen ist eine stromnetzunabhängige Spannungsversorgung bevorzugt, insbesondere mit Batterien, Akkumulatoren oder Solarzellen. Im Fall der stromnetzunabhängigen Spannungsversorgung kann ein Batterie- oder Akkumulatorfach für die Spannungsversorgung des Messgerätes vorgesehen sein, vorzugsweise auf der Rückseite des Messgerätes. Das Einschalten des Messgerätes kann automatisch erfolgen, beispielsweise durch das Einführen von Verbrauchsmittel, insbesondere eines Teststreifens, in das Gerät, oder durch das Auftragen des zu analysierenden fluiden Mediums auf einen bereits in das Gerät eingeführten Teststreifen. Die letztgenannte Variante bietet sich insbesondere bei Messgeräten an, die ein Aufnahmeelement zur Aufnahme der Verpackungseinheit für die Verbrauchsmittel (z.B. zur Aufnahme von Teststreifen-Magazinen) aufweisen. Das Abschalten kann ebenfalls automatisch erfolgen, beispielsweise beim Entfernen von benutztem Verbrauchsmittel, insbesondere eines benutzten Teststreifens, aus dem Gerät. Beide Funktionen können auch manuell, insbesondere durch Bedienen eines Schalters oder Tasters, auszuführen sein. Möglich ist auch, dass sowohl eine manuelle als auch automatische Bedienung möglich bzw. erforderlich ist. Vorzugsweise wird das Gerät automatisch eingeschaltet. Das Abschalten erfolgt vorzugsweise ebenfalls automatisch.

Das Anzeigeelement des tragbaren analytischen Messgerätes zum Anzeigen von Messergebnissen sollte eine gute Ablesbarkeit der angezeigten Daten gewährleisten. Als Anzeigeelement sind insbesondere Flüssigkristall-Displays (LCD), Leuchtelektroden (LED)-Displays und/oder organische Leuchtelektroden (OLED)-Displays geeignet. Die Displays können schwarz-weiß, in Grauabstufungen, einfarbig oder mehrfarbig ausgestaltet sein. Weiterhin ist eine Ausgestaltung als graphisches Pixel-Display möglich. Die Anzeige von Daten kann numerisch, graphisch, in Form von Piktogrammen, etc. erfolgen. Gegebenenfalls kann eine zusätzliche (Hintergrund-)Beleuchtung vorgesehen werden. Das Anzeigeelement ist insbesondere auf der Vorderseite des Messgerätes angeordnet.

Die erste Datenempfangsvorrichtung des tragbaren analytischen Messgerätes kann zum drahtlosen Empfang von Daten und/oder zum Datenempfang über elektrische Kontakte ausgestaltet sein.

Zum drahtlosen Datenempfang umfasst die erste Datenempfangsvorrichtung insbesondere mindestens ein Transponder-Lesemodul, welches geeignet ist, in einem Transponder gespeicherte Daten auszulesen, d. h. zu empfangen. Unter einem Transponder ist hierbei speziell ein Radiofrequenz-Identifikations-Tag, kurz RFID-Tag (RFID = Radio-Frequency-Identification), oder eine einen solchen RFID-Tag aufweisende Vorrichtung zu verstehen. RFID-Tags zeichnen sich dadurch aus, dass ihre Speicherzellen berührungslos mehrfach gelesen, verändert und neu beschrieben werden können. Es können jedoch auch definierte Speicherbereiche des RFID-Tags zum nur einmaligen Beschreiben mit Daten bestimmt sein. Ferner ist es möglich, den Zugriff auf definierte Speicherbereiche des RFID-Tags von einer vorherigen Authentifizierung abhängig zu machen. Ein geeigneter Transponder weist mindestens eine Antenne zum Übertragen von Daten und/oder Energie auf, beispielsweise eine auf ein Substrat aufgedruckte Spule aus leitfähigem Material, z. B. Metall, insbesondere eine Kupferspule, oder konduktive Polymere oder ein Polymer-Metall-Gemisch, insbesondere druckfähige Silberleitpaste. Dem Fachmann ist bekannt, dass die Antenne auch in anderen Bauformen realisiert werden kann, z.B. in üblicher Weise derart, dass eine Generierung von Dipolen stattfindet. Vorzugsweise ist ein von dem Transponder-Lesemodul der Datenempfangsvorrichtung des tragbaren analytischen Messgerätes auszulesender Transponder ausgestaltet, um Daten zu empfangen und zu senden. Des Weiteren weist der Transponder einen eigenen Speicher auf. Zweckmäßigerweise weist der Transponder in der Regel außerdem einen Mikroprozessor auf. Weiterhin kann der Transponder eine eigene Energiequelle aufweisen, beispielsweise eine Batterie oder einen Akku. In diesem Zusammenhang spricht man auch von NFC (Near Field Communication) oder von aktiven Transpondern. Alternativ oder zusätzlich kann der Transponder extern mit Energie versorgt werden, beispielsweise durch Empfang entsprechender elektromagnetischer Wellen. Solche elektromagnetischen Wellen können insbesondere von der Datenempfangsvorrichtung des tragbaren analytischen Messgerätes ausgesandt werden. Die so zur Verfügung gestellte Energie kann auch zum Nach- bzw. Wiederaufladen des Akkus verwendet werden.

Als elektromagnetische Wellen für den Austausch von Daten und Energie zwischen den erfindungsgemäß eingesetzten Datenempfangs- bzw. Datenübertragungsvorrichtungen und einem Transponder kommen grundsätzlich verschiedene Spektralbereiche des elektromagnetischen Spektrums in Betracht, etwa in einem Bereich von 30 kHz bis zu 50 GHz. Üblicherweise verwendet werden Wellen in den sogenannten ISM-Bändern (Industrie, Science, Medizin), bei denen es sich um frei verfügbare Frequenzbänder handelt. Insbesondere sind Wellen in einem Frequenzbereich von 30 -125 kHz (Niederfrequenzwellen), von 10 bis 15 MHz (Hochfrequenzwellen), von 850 - 950 MHz (Ultrahochfrequenzwellen) sowie von 1 bis 10 GHz (Mikrowellen) geeignet. Es können grundsätzlich jedoch auch andere Spektralbereiche des elektromagnetischen Spektrums genutzt werden, beispielsweise im infraroten Spektralbereich (das heißt mit Frequenzen von mehr als 1 THz), insbesondere im Bereich von 750 nm bis 10 µm. Die möglichen Distanzen der Datenübertragung bzw. Energieübertragung zwischen Transponder und Transponder-Lesemodul hängen von der baulichen Ausgestaltung ab und liegen für die Zwecke der vorliegenden Erfindung üblicherweise im Bereich von 0,01 cm bis zu 5 m, insbesondere von 0,1 cm bis 2 m, und speziell von 0,5 cm bis 1 m. Der Fachmann erkennt in diesem Zusammenhang ohne Weiteres, dass die erfindungsgemäß eingesetzten Datenempfangs- bzw. Datenübertragungsvorrichtungen entsprechend ihres jeweiligen konkreten Einsatzzweckes bzw. Einsatzortes auszuwählen sind. So sind z.B. für eine drahtlose Datenübertragung im Vertriebsbereich, etwa in einem Lager eines Groß- oder Zwischenhändlers, andere Anforderungen an die eingesetzten Datenempfangs- bzw. Datenübertragungsvorrichtungen zu stellen, als bei der direkten Distribution an einen Endverbraucher, etwa durch einen Apotheker oder Arzt. Die unterschiedlichen Anforderungen, z.B. bezüglich Distanz, Datenübertragungsrate, etc., werden in der Regel vom Transponder-Lesemodul geleistet. Allein aufgrund der gegenüber den Transponder-Lesemodulen deutlich höheren Stückzahlen der Transponder, die mit den Verbrauchsmitteln vertrieben werden, ist bei den Transpondern der bauliche Aufwand (Komponenten, Material, etc.) möglichst gering zu halten. Die hierbei zu berücksichtigenden Faktoren und Lösungen sind dem Fachmann hinlänglich bekannt und beispielsweise in Finkenzeller, Klaus, RFID-Handbuch, 2. Aufl., Carl Hanser Verlag, München 2000 beschrieben.

Es versteht sich für den Fachmann, dass die hierin gemachten Aussagen über drahtlose Datenübertragung, z.B. betreffend die RFID-Technik, in analoger Weise allgemein auf alle erfindungsgemäß eingesetzten Datenempfangs- bzw. Datenübertragungsvorrichtungen anzuwenden sind, selbst wenn nur Bezug auf die erste Datenempfangsvorrichtung des analytischen Messgerätes genommen wird. Es versteht sich für den Fachmann ferner, dass die hierin erfindungsgemäß zum drahtlosen Datenempfang bzw. zur drahtlosen Datenübertragung verwendeten Vorrichtungen, die ein Transponder-Lesemodul bzw. ein Transponder-Schreibmodul aufweisen, üblicherweise nicht als getrennte Vorrichtungen ausgestaltet sind. Vielmehr werden Transponder-Lesemodul und Transponder-Schreibmodul in der Regel in einer einzigen Gesamtvorrichtung realisiert. Beim Betrieb der Gesamtvorrichtung sind Lese- und Schreibmodul zumindest funktional unterscheidbar, auch wenn beim Empfang und bei der Übertragung von Daten mindestens teilweise dieselben Komponenten genutzt werden. Die hierin stets getroffene Unterscheidung zwischen Datenempfangs- und Datenübertragungsvorrichtungen bzw. Lese-/Schreibmodule erfolgt zum Zweck einer klaren Darstellung der erfindungswesentlichen Funktionen der Bestandteile des erfindungsgemäßen Analysesystems. Die Einzelheiten der Ausgestaltung eines Transponder-Lesemoduls bzw. Transponder-Schreibmoduls sind dem Fachmann hinreichend bekannt und beispielsweise in Finkenzeller, Klaus, RFID-Handbuch, 2. Aufl., Carl Hanser Verlag, München 2000 beschrieben. Das Transponder-Lesemodul, z.B. des analytischen Messgerätes, weist üblicherweise einen Schwingkreis auf, der eine spulenförmige Antenne und einen Parallelkondensator umfasst. An diesen Schwingkreis sind ein Hochfrequenz(HF)-Generator, ein Modulator, ein Entkoppelfilter und ein Demodulator geschaltet. Vorzugsweise liefert in diesem Fall die erste Datenempfangsvorrichtung bzw. das Transponder-Lesemodul die Energie für den Transponder. Auf diese Weise können Daten aus dem Transponder ausgelesen werden. Um Daten auf den Transponder zu übertragen, kann das Messgerät eine entsprechende Datenübertragungsvorrichtung, insbesondere ein Transponder-Schreibmodul, aufweisen.

Um die erste Datenempfangsvorrichtung des tragbaren analytischen Messgerätes zur Energieversorgung eines Transponders mittels elektromagnetischer Wellen zu nutzen, kann die erste Datenempfangsvorrichtung eine Sendevorrichtung aufweisen, welche zunächst über ein drahtloses Signal den Transponder, der zum Beispiel Bestandteil des beschreibbaren nichtflüchtigen Datenspeichers ist, zum drahtlosen Übertragen von Daten anregt. Dieses Signal kann von der ersten Datenempfangsvorrichtung beispielsweise in regelmäßigen Abständen ausgesandt werden oder dann, wenn eine Öffnung oder ein Aufnahmeelement im Gerätegehäuse mit einem Verschluss geschlossen wird. Letzteres ist insbesondere vorteilhaft, wenn die Verpackungseinheit für die Verbrauchsmittel in ein entsprechendes Aufnahmeelement des Messgerätes eingeführt wird. Anschließend werden diese Daten mittels des Transponder-Lesemoduls der ersten Datenempfangsvorrichtung von dem tragbaren analytischen Messgerät empfangen. Das hier beschriebene Prinzip der Anregung eines Transponders zur Datenübertragung kann auch vorteilhaft genutzt werden, um automatisch eine drahtlose Datenübertragung zu starten, sobald eine ausreichende räumliche Nähe zwischen einer Datenempfangs- bzw. Übertragungsvorrichtung und einem Transponder besteht. Auf diese Weise kann z.B. die erste Datenempfangsvorrichtung automatisch dazu veranlasst werden, Daten von einem in der Nähe befindlichen Transponder zu empfangen. Analog kann eine Datenübertragungsvorrichtung automatisch dazu veranlasst werden, Daten auf einen in der Nähe befindlichen Transponder zu übertragen. Vorzugsweise gelangt ein Transponder dadurch in ausreichende räumliche Nähe zu der ersten Datenempfangsvorrichtung, indem die Verpackungseinheit mit dem zugeordneten beschreibbaren nichtflüchtigen Datenspeicher, der den Transponder umfasst, einem Aufnahmeelement zur Aufnahme der Verpackungseinheit oder einer Positionierungshilfe des tragbaren analytischen Messgerätes zugeführt wird.

Zum Datenempfang über elektrische Kontakte umfasst die erste Datenempfangsvorrichtung des tragbaren analytischen Messgerätes insbesondere ein Lesemodul, welches geeignet ist, Daten aus einem elektronischen Speicherelement, z. B. einem Read-Only-Memory (ROM)-Baustein, einem EEPROM-Speicherelement oder einem Flash-Speicherelement, auszulesen. Hierbei umfasst die erste Datenempfangsvorrichtung weiterhin insbesondere eine elektrische Kupplung zur Herstellung des elektrischen Kontakts zwischen dem Messgerät und dem beschreibbaren Datenspeicher. Es versteht sich für den Fachmann, dass die hierin gemachten Aussagen über verdrahtete Datenübertragung (d.h. über elektrische Kontakte) in analoger Weise allgemein auf alle erfindungsgemäß eingesetzten Datenempfangs- bzw. Datenübertragungsvorrichtungen anzuwenden sind, selbst wenn nur Bezug auf die erste Datenempfangsvorrichtung des analytischen Messgerätes genommen wird.

Der Begriff "beschreibbarer nichtflüchtiger Datenspeicher" bedeutet im Sinne der vorliegenden Erfindung, dass der Datenspeicher mindestens einen Speicherbereich aufweist, der mindestens einmalig und insbesondere mehrfach, d. h. mehr als einmalig, mit Daten, vorzugsweise digitalen Daten, beschreibbar ist. Die Daten können beliebig oft ausgelesen werden. Prinzipiell kommen Speicherelemente und Speicherbausteine auf Basis (elektro-)magnetischer oder biologischer Datenspeicherung, auf Halbleiterbasis oder auf Basis konduktiver Polymere in Betracht. Im Sinne der vorliegenden Erfindung werden einmalig beschreibbare Datenspeicher als ROM-Bausteine bezeichnet. ROM-Bausteine werden generell vom Hersteller programmiert, z.B. über Maskenprozesse, und weisen somit einen vordefinierten Speicherinhalt auf. Dieser Speicherinhalt kann aber auch vom Kunden bzw. Anwender vorgegeben werden. Einmalig beschreibbare Speicherbereiche werden üblicherweise auch als OTP (one-time-programmable) oder als PROM (programmable read-only-memory) bezeichnet. EEPROM-Speicherelemente und Flash-Speicherelemente hingegen sind elektrisch mehrfach, d.h. wiederholt, beschreibbare und löschbare Datenspeicher.

Für die Zwecke der vorliegenden Erfindung sind insbesondere Datenspeicher geeignet, die mindestens einen mehrfach beschreibbaren Speicherbereich aufweisen, z. B. ein EEPROM-Speicherelement und/oder ein Flash-Speicherelement. Bevorzugt ist ein Transponder, der ein RFID-Tag, wie zuvor beschrieben, umfasst. Speziell geeignet sind Datenspeicher, die sowohl einen einmalig beschreibbaren Speicherbereich, z. B. einen ROM-Baustein, als auch einen wiederholt beschreibbaren Speicherbereich, z. B. ein EEPROM-Speicherelement, Flash-Speicherelement und/oder einen Transponder, insbesondere mit RFID-Tag, wie zuvor beschrieben, aufweisen. Besonders bevorzugt ist ein Transponder mit RFID-Tag. In der Regel besitzt ein solcher RFID-tag einen Speichertyp, üblicherweise ein EEPROM-Speicherelement oder ein Flash-Speicherelement, der jedoch über mehrere verschiedene Speicherbereiche verfügt. Die Festlegung, welche Speicherbereiche nur einfach beschreibbar sind (OTP) bzw. welche Speicherbereiche mehrfach beschreibbar sind, kann insbesondere über Software erfolgen. In Betracht kommen ferner RAM-Bausteine, die jedoch eine permanente Spannungsversorgung benötigen, um nichtflüchtig zu sein (NFC).

Der beschreibbare nichtflüchtige Datenspeicher kann weiterhin ein Authentifizierungselement umfassen, durch welches ein Auslesen und/oder Beschreiben einzelner oder aller Speicherbereiche des beschreibbaren nichtflüchtigen Datenspeichers erst nach erfolgreicher Authentifizierung autorisiert wird. Hierdurch wird einerseits ein unautorisierter Zugriff auf die gespeicherten Daten und andererseits eine unautorisierte Veränderung der gespeicherten Daten verhindert. Derartige Authentifizierungselemente bzw. Verfahren zur Authentifizierung sind dem Fachmann bekannt und z.B. in Finkenzeller, Klaus, RFID-Handbuch, 2. Aufl., Carl Hanser Verlag, München 2000 beschrieben. Üblicherweise erfordert eine erfolgreiche Authentifizierung, dass die jeweilige Datenempfangs- bzw. Datenübertragungsvorrichtung zum Auslesen bzw. Beschreiben des jeweiligen Speicherbereichs über eine Sollinformation, z.B. einen Softwareschlüssel, einen Code oder ein Passwort, verfügt, die von dem Authentifizierungselement erkannt, d.h. verifiziert, wird.

In einer bevorzugten Ausführungsform umfasst der beschreibbare nichtflüchtige Datenspeicher ein Authentifizierungselement, durch welches ein Auslesen und/oder Beschreiben mindestens eines der Speicherbereiche des beschreibbaren nichtflüchtigen Datenspeichers erst nach erfolgreicher Authentifizierung autorisiert wird. Besonders bevorzugt ist, dass ein Beschreiben aller Speicherbereiche des Datenspeichers erst nach erfolgreicher Authentifizierung autorisiert wird. Darüber hinaus kann der Datenspeicher auch so eingerichtet sein, dass eine erfolgreiche Authentifizierung zum Auslesen und Beschreiben aller Speicherbereiche des Datenspeichers erforderlich ist.

In einer weiteren bevorzugten Ausführungsform der vorliegenden Erfindung weist der beschreibbare nichtflüchtige Datenspeicher einen ersten Speicherbereich auf, der nur einmal mit Daten beschreibbar ist, und einen zweiten Speicherbereich, der mindestens einmal und vorzugsweise mehrfach mit Daten beschreibbar ist. Hierbei weist der Datenspeicher gegebenenfalls weiterhin einen dritten Speicherbereich auf, der mindestens einmal und vorzugsweise mehrfach beschreibbar ist. Es versteht sich, dass der beschreibbare nichtflüchtige Datenspeicher, sofern gewünscht, weitere Speicherbereiche aufweisen kann. Besonders bevorzugt ist hierbei die erste Datenübertragungsvorrichtung zum Übertragen der Steuerungsdaten in den ersten Speicherbereich des beschreibbaren nichtflüchtigen Datenspeichers eingerichtet. Gleichermaßen besonders bevorzugt ist hierbei die zweite Datenübertragungsvorrichtung zum Übertragen der Anzeigedaten in den zweiten Speicherbereich des beschreibbaren nichtflüchtigen Datenspeichers eingerichtet. Ganz besonders bevorzugt umfasst der beschreibbare nichtflüchtige Datenspeicher hierbei ein Authentifizierungselement, durch welches ein Auslesen und/oder Beschreiben des zweiten Speicherbereichs des beschreibbaren nichtflüchtigen Datenspeichers erst nach erfolgreicher Authentifizierung autorisiert wird. Gleichermaßen besonders bevorzugt ist hierbei der dritte Speicherbereich des Datenspeichers ohne vorherige Authentifizierung beschreibbar.

Der beschreibbare nichtflüchtige Datenspeicher kann zum drahtlosen Empfangen von Daten und/oder zum Empfangen von Daten über einen elektrischen Kontakt ausgestaltet sein. Für die drahtlose Übertragung weist der beschreibbare nichtflüchtige Datenspeicher insbesondere mindestens einen Transponder auf. Der Transponder kann dabei so ausgestaltet sein wie zuvor beschrieben, mit der Maßgabe, dass er zum Senden und Empfangen von Daten ausgestaltet ist. Für die Übertragung von Daten über einen elektrischen Kontakt weist der beschreibbare nichtflüchtige Datenspeicher insbesondere ein Speicherelement auf, welches üblicherweise über eine Drahtverbindung kontaktiert werden kann, z.B. ein EEPROM-Speicherelement, Flash-Speicherelement und/oder ROM-Baustein.

Vorzugsweise weist der beschreibbare nichtflüchtige Datenspeicher mindestens einen Speicherbereich auf, in den Daten drahtlos übertragen werden können. Hierbei kann der Datenspeicher zusätzlich mindestens einen Speicherbereich aufweisen, in den Daten über einen elektrischen Kontakt übertragen werden können. In diesem Fall stehen die Speicherbereiche vorzugsweise derart miteinander in Kontakt, dass auf alle gespeicherten Daten sowohl drahtlos als auch über einen elektrischen Kontakt zugegriffen werden kann. Eine derartige Anordnung der Speicherbereiche des beschreibbaren nichtflüchtigen Datenspeichers kann z.B. in einem (drahtlos ansprechbaren) Transponder realisiert werden, der einerseits mit einem elektronischen Speicherelement ausgestattet ist und andererseits elektrische Kontakte aufweist. Hierfür geeignete sogenannte Dual-Port-EEPROM-Speicherelemente sind bereits kommerziell verfügbar, z.B. von der Firma ATMEL (ATMEL AT24RF08C). Besonders bevorzugt ist es jedoch, dass der beschreibbare nichtflüchtige Datenspeicher drahtlos beschrieben und/oder ausgelesen werden kann. Hierbei ist es ganz besonders bevorzugt, dass alle Speicherbereiche des Datenspeichers drahtlos beschrieben und/oder ausgelesen werden können.

Vorzugsweise weist die erste Datenempfangsvorrichtung des analytischen Messgerätes ein Transponder-Lesemodul zum drahtlosen Datenempfang auf. Gegebenenfalls weist die erste Datenempfangsvorrichtung hierbei zusätzlich ein Lesemodul zum Datenempfang über elektrische Kontakte auf. In diesem Fall können in einem Speicherelement des Transponders, z.B. einem EEPROM-Speicherelement, einem Flash-Speicherelement oder einem ROM-Baustein, gespeicherte Daten auch über eine Drahtverbindung ausgelesen werden.

Die erste Datenübertragungsvorrichtung ist erfindungsgemäß zum Übertragen der Steuerungsdaten auf den beschreibbaren nichtflüchtigen Datenspeicher eingerichtet. Zu diesem Zweck umfasst sie üblicherweise mindestens ein Schreibmodul zum drahtlosen oder verdrahteten (d.h. über einen elektrischen Kontakt) Übertragen von Daten, insbesondere der Steuerungsdaten. In einer bevorzugten Ausführungsform weist die erste Datenübertragungsvorrichtung ein Transponder-Schreibmodul zum drahtlosen Übertragen von Daten, insbesondere der Steuerungsdaten, auf. Hierbei kann die erste Datenübertragungsvorrichtung zusätzlich ein Schreibmodul aufweisen, welches Daten über einen elektrischen Kontakt übertragen kann. In einer weiteren bevorzugten Ausführungsform weist die erste Datenübertragungsvorrichtung ein Schreibmodul zum Übertragen von Daten über einen elektrischen Kontakt auf (ohne dass ein Schreibmodul zum drahtlosen Übertragen von Daten vorgesehen ist).

Die zweite Datenübertragungsvorrichtung ist erfindungsgemäß zum Übertragen der Anzeigedaten auf den beschreibbaren nichtflüchtigen Datenspeicher eingerichtet. Zu diesem Zweck umfasst sie üblicherweise mindestens ein Schreibmodul zum drahtlosen oder verdrahteten (d.h. über einen elektrischen Kontakt) Übertragen von Daten, insbesondere der Anzeigedaten. In einer bevorzugten Ausführungsform weist die zweite Datenübertragungsvorrichtung ein Transponder-Schreibmodul zum drahtlosen Übertragen von Daten, insbesondere der Anzeigedaten, auf. Hierbei kann die zweite Datenübertragungsvorrichtung zusätzlich ein Schreibmodul aufweisen, welches Daten über einen elektrischen Kontakt übertragen kann. Ganz besonders bevorzugt weist die zweite Datenübertragungsvorrichtung ein Schreibmodul zum drahtlosen Übertragen von Daten auf (ohne dass ein Schreibmodul zum drahtlosen Übertragen von Daten vorgesehen ist).

Das analytische Messgerät kann eine eigene Datenübertragungsvorrichtung (hierin als dritte Datenübertragungsvorrichtung bezeichnet) zum Übertragen von Daten, insbesondere von Messergebnissen und/oder die Messung betreffenden Messdaten, auf den beschreibbaren nichtflüchtigen Datenspeicher aufweisen. Zu diesem Zweck umfasst sie üblicherweise mindestens ein Schreibmodul zum drahtlosen oder verdrahteten (d.h. über einen elektrischen Kontakt) Übertragen von Daten, insbesondere der vorgenannten Messergebnisse bzw. Messdaten. Insbesondere weist die dritte Datenübertragungsvorrichtung ein Transponder-Schreibmodul zum drahtlosen Übertragen von Daten, insbesondere der Messergebnisse bzw. Messdaten, auf. Alternativ oder zusätzlich kann die dritte Datenübertragungsvorrichtung ein Schreibmodul zum Übertragen von Daten, insbesondere der Messergebnisse bzw. Messdaten, über einen elektrischen Kontakt aufweisen. Die dritte Datenübertragungsvorrichtung ist vorzugsweise eingerichtet, um die Messergebnisse und/oder Messdaten auf einen Speicherbereich des beschreibbaren nichtflüchtigen Datenspeichers zu übertragen, der ein Authentifizierungselement umfasst, durch welches ein Auslesen und/oder Beschreiben dieses Speicherbereichs erst nach erfolgreicher Authentifizierung autorisiert wird. Zu beachten ist, dass die dritte Datenübertragungsvorrichtung, sofern vorhanden, Bestandteil des tragbaren analytischen Messgerätes ist-dies gilt jedoch nicht für die erfindungsgemäß vorgesehene erste und zweite Datenübertragungsvorrichtung. Letztere kommen unabhängig von dem analytischen Messgerät zum Einsatz, wie weiter unten ausführlicher dargelegt wird.

Wenn das tragbare analytische Messgerät eine dritte Datenübertragungsvorrichtung zum Übertragen von Daten aufweist, so umfasst das Analysesystem vorzugsweise eine weitere Datenempfangsvorrichtung (hierin als zweite Datenempfangsvorrichtung bezeichnet) zum Auslesen von auf dem beschreibbaren Datenspeicher gespeicherten Daten, insbesondere der Messergebnisse bzw. Messdaten. Die zweite Datenempfangsvorrichtung weist üblicherweise mindestens ein Lesemodul zum drahtlosen oder verdrahteten (d.h. über einen elektrischen Kontakt) Empfang von Daten, insbesondere der vorgenannten Messergebnisse bzw. Messdaten, auf. Insbesondere weist die zweite Datenempfangsvorrichtung ein Transponder-Lesemodul zum drahtlosen Empfang von Daten, insbesondere der Messergebnisse bzw. Messdaten, auf.

In einer bevorzugten Ausführungsform der vorliegenden Erfindung weist der beschreibbare nichtflüchtige Datenspeicher einen Transponder, wie zuvor beschrieben, auf; die erste Datenübertragungsvorrichtung weist ein Transponder-Schreibmodul auf; die zweite Datenübertragungsvorrichtung weist ebenfalls ein Transponder-Schreibmodul auf; und die erste Datenempfangsvorrichtung des tragbaren analytischen Messgerätes weist ein Transponder-Lesemodul auf. Sofern das Messgerät eine dritte Datenübertragungsvorrichtung aufweist, so umfasst diese hierbei ebenfalls ein Transponder-Schreibmodul.

In einer weiteren bevorzugten Ausführungsform weist der beschreibbare nichtflüchtige Datenspeicher einen Transponder, wie zuvor beschrieben, der auch elektrisch kontaktiert werden kann, auf; die erste Datenübertragungsvorrichtung weist ein Schreibmodul zum Übertragen von Daten, insbesondere der Steuerungsdaten, über elektrische Kontakte auf; die zweite Datenübertragungsvorrichtung weist ein Transponder-Schreibmodul zum drahtlosen Übertragen von Daten, insbesondere der Anzeigedaten, auf; und die erste Datenempfangsvorrichtung des tragbaren analytischen Messgerätes weist ein Transponder-Lesemodul zum drahtlosen Datenempfang und/oder ein Lesemodul zum Datenempfang über elektrische Kontakte auf. Sofern das Messgerät eine dritte Datenübertragungsvorrichtung aufweist, so umfasst diese hierbei ein Transponder-Schreibmodul zur drahtlosen Datenübertragung und/oder ein Schreibmodul zur Datenübertragung über elektrische Kontakte.

Bei den Verbrauchsmitteln, die von dem tragbaren analytischen Messgerät zur Messung einer Analytkonzentration in einem fluiden Medium verwendet werden, handelt es sich insbesondere um Teststreifen oder andere Testelemente, wie kleine Teströhrchen oder kontinuierliche Rollen von Teststreifen. Solche Teststreifen sind beispielsweise für eine elektrochemische und/oder photometrische Analyse flüssiger Proben geeignet. Solche Teststreifen können zum Beispiel ein Kapillarsystem und eine oder mehrere Elektroden sowie ggf. zusätzliche chemische Hilfsstoffe, wie Enzyme und Mediatoren, aufweisen. Die Messung beruht hierbei auf einem amperometrischen Messprinzip. Üblich sind weiterhin Teststreifen, die einen schichtartigen Aufbau auf einem festen Substrat besitzen, wobei die infolge einer chemischen Reaktion innerhalb einer oder mehrerer der Schichten auftretende farbliche Veränderung photometrisch detektiert werden kann. Zum Einsatz in der vorliegenden Erfindung sind insbesondere Teststreifen geeignet, welche die Messung einer Analytkonzentration, insbesondere Glukose, in einem fluiden Medium, insbesondere in einer Körperflüssigkeit wie Blut oder interstitielle Flüssigkeit, erlauben.

Weiterhin können erfindungsgemäß Verbrauchsmittel zum Einsatz kommen, die nicht direkt von dem tragbaren analytischen Messgerät zur Messung einer Analytkonzentration in einem fluiden Medium verwendet werden, sondern die zur Vorbereitung einer solchen Messung verwendet werden. Hierzu zählen z.B. Sensoren zur Durchführung einzelner oder fortdauernder Messungen der Analytkonzentration in einem Körpergewebe und Lanzetten zum Hervorbringen von Körperflüssigkeiten wie Blut oder interstitielle Flüssigkeit aus Körpergewebe. Unter "fortdauernden Messungen" sind hierin kontinuierliche, z.B. über einen Zeitraum von einer Woche, und in Abständen wiederholt stattfindende Messungen zu verstehen. Insbesondere sind hierbei in die Haut insertierbare (Glukose-)Sensoren, wie zuvor beschrieben, umfasst. Sofern nicht anders angegeben, sind hierin in der Regel sowohl die in diesem Absatz als auch die in dem vorhergehenden Absatz beschriebenen Verbrauchsmittel gemeint, wenn der Ausdruck Verbrauchsmittel verwendet wird.

In einer bevorzugten Ausführungsform der vorliegenden Erfindung sind die Verbrauchsmittel ausgewählt unter Teststreifen zur Durchführung der Messung der Analytkonzentration, Sensoren zur Durchführung fortdauernder Messungen der Analytkonzentration in einem Körpergewebe, und Lanzetten zum Hervorbringen von Körperflüssigkeiten aus Körpergewebe.

Die Verbrauchsmittel werden zum Zwecke der Distribution bzw. des Verkaufs in Behältnisse zur sicheren Aufbewahrung, wie Röhrchen oder Dosen aus Metall, Glas, Kunststoff und/oder Verbundwerkstoffen gegeben. Diese Behältnisse können ihrerseits in Umverpackungen, beispielsweise aus Papier, Karton, Kunststoff und/oder Metall verpackt vorliegen. Die Verpackungseinheit für die Verbrauchsmittel ist insbesondere ausgewählt unter Behältnissen zur magazinierten Aufbewahrung mehrerer der Verbrauchsmittel (hierin als Verbrauchsmittel-Magazine bezeichnet), sowie unter Umverpackungen für derartige Behältnisse. Beispiele für geeignete Behältnisse sind insbesondere Trommel-Magazine, Stangen-Magazine und Reihen-Magazine. Im Fall von z.B. Teststreifen zur Bestimmung des Glukosegehaltes in Blut werden üblicherweise 10 bis 250 Teststreifen, insbesondere 50 Teststreifen in eine Verpackungseinheit, z. B. ein Kunststoff- oder Metallröhrchen, gegeben. Als Einzelteststreifen kommen unter Anderem auch solche mit Transponder in Betracht, siehe WO 2006/040083 A1. Auch andere Arten der Magazinierung der Verbrauchsmittel sind denkbar, insbesondere Bandkassetten mit einer Vielzahl auf einem kontinuierlichen Träger, z.B. einer flexiblen Rolle, angeordneter Verbrauchsmittel. Die Zuordnung des beschreibbaren nichtflüchtigen Datenspeichers zu der Verpackungseinheit kann erfindungsgemäß ausgestaltet sein, indem der Datenspeicher fest mit der Verpackungseinheit verbunden angeordnet wird, beispielsweise indem er mit dieser verklebt wird. Alternativ kann der beschreibbare nichtflüchtige Datenspeicher der Verpackungseinheit so beigefügt sein, dass er aus dieser auf einfache Weise entnehmbar ist, insbesondere indem der Datenspeicher lose in ein Behältnis, zum Beispiel ein Kunststoff- oder Metallröhrchen oder ein anderes Verbrauchsmittel-Magazin, oder in eine Umverpackung, zum Beispiel aus Papier, Pappe, Metall und/oder Kunststoff, eingelegt wird. Bevorzugt ist als Verpackungseinheit für die Verbrauchsmittel ein Behältnis zur magazinierten Aufbewahrung mehrerer der Verbrauchsmittel vorgesehen, wobei die Behältnisse insbesondere ausgewählt sind unter Trommel-Magazinen, Stangen-Magazinen, Reihen-Magazinen und Bandkassetten.

Des Weiteren kann das tragbare analytische Messgerät einen Testzähler aufweisen. Der Testzähler kann insbesondere vorteilhaft im Fall von Verbrauchsmittel-Magazinen, eingesetzt werden. Mit dem Testzähler können z.B. die in einem Magazin vorhandenen unverbrauchten Verbrauchsmittel ermittelt und gegebenenfalls auf dem Anzeigeelement des Messgerätes angezeigt werden. Eine solche Erfassung der verbrauchten bzw. unverbrauchten Verbrauchsmittel erfolgt, indem der Testzähler entweder dekrementiert zählt, d.h. von einer Gesamtanzahl N bis auf 0 Verbrauchsmittel herunter, oder inkrementiert zählt, d.h. von 0 auf eine Gesamtanzahl N hinauf. Zu dem jeweiligen Zählerstand für ein bestimmtes Verbrauchsmittel können korrespondierende Daten gespeichert werden, z.B. Messwerte, Betriebsspannungen, Temperaturen, etc. Diese Daten können insbesondere im Fall einer Reklamation durch den Endanwender des Messgerätes ausgewertet werden. Zweckmäßig ist das analytische Messgerät so ausgestaltet, dass es die Messung betreffende Daten automatisch dem jeweiligen Testzählerstand zuordnet. Hierbei wird in der Regel für jeden Zählerstand ein separater Datensatz angelegt und gespeichert. Der Zählerstand kann vorteilhafterweise auf dem der Verpackungseinheit für die Verbrauchsmittel zugeordneten beschreibbaren, nichtflüchtigen Datenspeicher abgelegt werden. Dies ermöglicht den bequemen Einsatz der Verbrauchsmittel in anderen Messgeräten.

Unter Steuerungsdaten im Sinne der vorliegenden Erfindung sind alle Daten zu verstehen, die geeignet sind, die Durchführung der analytischen Funktionen des tragbaren analytischen Messgerätes einschließlich eventueller Softwarefunktionen zu ermöglichen bzw. zu verbessern. Somit können die Steuerungsdaten Funktionen betreffen, die mit der Vorbereitung und Durchführung einer Messung, dem Aufzeichnen und Sammeln von Messdaten sowie dem Auswerten und Übertragen von Messdaten bzw. Messergebnissen in Zusammenhang stehen, insbesondere die Umwandlung von Messdaten in Messergebnisse unter Verwendung chargenspezifischer Kenndaten zur Kalibrierung. Chargenspezifische Kenndaten sind häufig erforderlich, um produktionsbedingte Schwankungen in der Nachweischarakteristik der eingesetzten Verbrauchsmittel, insbesondere von Teststreifen, wie Teststreifen zur Bestimmung von Blutglukosekonzentrationen, auszugleichen. Die Erstellung chargenspezifischer Kenndaten (Steuerungsdaten) erfolgt üblicherweise so, dass eine Charge der Verbrauchsmittel hergestellt wird, von dieser Charge eine Teilmenge entnommen wird, und anhand der entnommenen Teilmenge werden chargenspezifische Steuerungsdaten gewonnen. Diese chargenspezifischen Steuerungsdaten werden erfindungsgemäß mittels der ersten Datenübertragungsvorrichtung auf den beschreibbaren nichtflüchtigen Datenspeicher übertragen, wobei der Datenspeicher vor oder nach dem Übertragen der chargenspezifischen Steuerungsdaten der Verpackungseinheit zugeordnet wird.

Vorzugsweise umfassen die Steuerungsdaten Reparaturdaten, welche die Korrektur fehlerhafter Funktionen des analytischen Messgerätes ermöglichen und/oder Upgrade-Daten, welche eine Aktualisierung der Funktionen des analytischen Messgerätes ermöglichen. Solche Reparatur- bzw. Upgradedaten umfassen insbesondere Daten zur Korrektur fehlerhafter Softwarefunktionen bzw. Daten zur Aktualisierung von Softwarefunktionen für eine Software, die von dem tragbaren analytischen Messgerät verwendet wird. Eine solche Software kann insbesondere Gerätefunktionen im Zusammenhang mit der Durchführung von Messungen mit dem analytischen Messgerät steuern bzw. kontrollieren. Weiterhin kann eine solche Software auch die Auswertung der (physikalischen) Messwerte oder die Anzeige von Messergebnissen auf dem Anzeigeelement kontrollieren. So kann z.B. die Darstellung der zuvor genannten Daten (z. B. Messergebnisse) auf einem Display des Messgerätes an individuelle Bedürfnisse eines Anwenders angepasst werden, ohne dass hierfür zusätzliche Bedienhandlungen seitens des Anwenders erforderlich sind. Es können dieselben Daten beispielsweise mit unterschiedlicher Schriftgröße, Zeichendichte, Zeilenabstand, Menge der zeitgleich angezeigten Informationen, etc. wunschgemäß oder auch altersgruppengerecht angezeigt werden. Mit Steuerungsdaten können z.B. bestimmte analytische Messegräte gezielt deaktiviert werden, insbesondere wenn Produktionsfehler in einzelnen Chargen auftreten, so dass auf diese Weise Fehlmessungen unterbunden werden können.

Bei den Steuerungsdaten handelt es sich im Allgemeinen um sogenannte non-evidente Daten, die dem Anwender nicht zugänglich sind, insbesondere nicht auf dem Anzeigeelement des tragbaren analytischen Messgerätes angezeigt werden. Derartige Steuerungsdaten werden in der Regel nur von authorisierten Stellen, z.B. dem Hersteller von Testreifen, erstellt und gegebenenfalls verändert, sofern letzteres überhaupt technisch vorgesehen ist (z.B. wenn die Steuerungsdaten in einem mehr als einmal beschreibbaren Speicherbereich des beschreibbaren nichtflüchtigen Datenspeichers gespeichert werden). Vorzugsweise werden die Steuerungsdaten daher in einem Speicherbereich des beschreibbaren nichtflüchtigen Datenspeichers, der nur einmal mit Daten beschreibbar ist, gespeichert. Alternativ kann es zweckmäßig sein, die Steuerungsdaten in einem mehrfach beschreibbaren Speicherbereich des Datenspeichers zu speichern und diesen Speicherbereich mittels eines Authentifizierungselementes oder Authentifizierungsverfahrens, wie zuvor beschrieben, gegen nicht authorisierten Zugriff zu schützen.

Im Unterschied zu den Steuerungsdaten handelt es sich bei den Anzeigedaten im Sinne der vorliegenden Erfindung um Daten, die zumindest teilweise auf dem Anzeigeelement zum Anzeigen von Messergebnissen des tragbaren analytischen Messgerätes angezeigt werden. Hierbei kann es sich um alle Arten von Daten handeln, die für den Anwender von Interesse sein können, beispielsweise im Zusammenhang mit der praktischen Durchführung von Messungen mit dem tragbaren analytischen Messgerät und/oder allgemeine Informationen im Zusammenhang mit der Distribution der Verbrauchsmittel. So können die Anzeigedaten z. B. Öffnungszeiten, Kontaktdaten, Angaben zu Serviceleistungen, etc. von Ärzten und/oder Apothekern umfassen, wobei derartige Anzeigedaten lokal und/oder zeitlich unterschiedlich ausgestaltet sein können. Weiterhin können die Anzeigedaten patientenspezifische Anweisungen, insbesondere von einem Arzt, Apotheker und/oder einer Krankenschwester, zur Durchführung von Messungen mittels des tragbaren analytischen Messgerätes umfassen, beispielsweise Anweisungen betreffend die Zeit, Dauer und/oder Häufigkeit der Durchführung von Messungen mit Hilfe des tragbaren analytischen Messgerätes.

In einer weiteren bevorzugten Ausführungsform ist die erste Datenübertragungsvorrichtung in einer Organisatonseinheit gemeinsam mit der Erstellung der Steuerungsdaten, insbesondere chargenspezifischer Kenndaten, zusammengefasst. Dies kann z. B. durch eine gemeinsame räumlich und/oder zeitlich nahe Anordnung realisiert werden. Räumliche bzw. zeitliche Nähe bedeutet in diesem Zusammenhang, dass chargenspezifische Steuerungsdaten, die anhand einer von einer produzierten Charge (der Verbrauchsmittel) entnommenen Teilmenge gewonnen wurden, möglichst direkt auf den beschreibbaren Datenspeicher übertragen werden und dieser der Verpackungseinheit der jeweiligen Charge zugeordnet wird. Hierbei sollten keine längeren Zeiten, z. B. mehrtägige Lagerungszeiträume, zwischen der Erstellung der Steuerungsdaten und deren Übertragung auf den beschreibbaren nichtflüchtigen Datenspeicher und/oder dessen Zuordnung zu der Verpackungseinheit auftreten. Vorteilhaft ist hierbei, wenn die Verbrauchsmittel nach ihrer Herstellung möglichst direkt in ihre endgültige Verpackung bzw. Verpackungseinheit überführt werden. Auf diese Weise kann insbesondere eine Beeinträchtigung infolge des Einflusses von Feuchtigkeit oder Luft ausgeschlossen werden. Hierbei ist es bevorzugt, dass der beschreibbare nichtflüchtige Datenspeicher einen Transponder, insbesondere mit RFID-Tag, umfasst. In diesem Fall können nämlich die Steuerungsdaten auch nach Zuordnung des Datenspeichers zu der Verpackungseinheit (drahtlos) auf den Datenspeicher übertragen werden, ohne dass die Verpackung bzw. Verpackungseinheit erneut geöffnet wird.

In einer besonders bevorzugten Ausführungsform ist die erste Datenübertragungsvorrichtung Bestandteil eines werksseitigen Systems, wobei das werksseitige System
- die Herstellung und Verpackung der Verbrauchsmittel in Verpackungseinheiten; und
- die Zuordnung des beschreibbaren nichtflüchtigen Datenspeichers zu den Verpackungseinheiten umfasst, und wobei die zweite Datenübertragungsvorrichtung Bestandteil eines werksfernen Systems ist, wobei das werksferne System
- eine Vertriebsstelle zum Handel mit den Verpackungseinheiten, und/oder
- eine Distributionsstelle zur Distribution der Verpackungseinheiten an einen Endverbraucher umfasst.

Als Vertriebsstelle bzw. Distributionsstelle im Sinne der vorliegenden Erfindung kommen alle Stellen in Betracht, die im Anschluss an die werksseitige Auslieferung der Verbrauchsmittel mit deren Handel, Vertrieb, Verteilung, Ausgabe und/oder Verkauf an (Zwischen-)Händler und/oder an Endverbraucher, insbesondere an Endverbraucher, befasst sind. Eine Distributionsstelle kann zum Beispiel ein Apotheker, Arzt und/oder eine Krankenschwester sein. Die Verwendung der Begriffe Apotheker, Arzt bzw. Krankenschwester soll im Sinne der vorliegenden Erfindung den räumlichen Wirkungsbereich des Arztes, Apothekers bzw. der Krankenschwester umfassen, insbesondere also eine Arztpraxis, eine Apothekenniederlassung bzw. eine Krankenhausabteilung. Der Arzt oder Apotheker bzw. die Krankenschwester kann jedoch auch am Aufenthaltsort des Anwenders bzw. Patienten tätig werden, beispielsweise am Wohnort des Anwenders oder in einem Krankenhaus, wobei in diesem Fall unter der Distributionsstelle der Ort des Tätigwerdens des Arztes oder Apothekers bzw. der Krankenschwester zu verstehen ist. Ein wesentlicher Aspekt dieser Ausführungsform besteht darin, dass das werksseitige System und das werksferne System eine klare organisatorische Trennung, insbesondere eine räumliche Trennung, voneinander aufweisen. In der Regel wird zwischen dem werksseitigen System und dem werksfernen System ein Vertriebssystem zwischengeschaltet sein, mittels dessen die Verbrauchsmittel, die im werksseitigen System hergestellt, verpackt und mit dem Datenspeicher versehen wurden, der Distributionsstelle des werksfernen Systems zugeführt werden. Ein solches Vertriebssystem erfolgt insbesondere über verschiedene Händler, z.B. Groß- und/oder Zwischenhändler.

Zur Ausgestaltung konkreter Arbeitsabläufe ist zwischen dem werksseitigen und dem werksfernen System üblicherweise eine organisatorische Kopplung vorzusehen, wobei insbesondere die jeweiligen Schreibrechte zu regeln sind. Die technische Realisierung kann hierbei vorteilhaft über eigens vorzusehende Übertragungsvorrichtungen (z.B. Internet, Telefon, Softwareeinsatz) zwischen den beiden Systemen erfolgen.

In einer weiteren Ausführungsform ist die erste Datenempfangsvorrichtung eingerichtet, um ohne eigene Spannungsquelle drahtlos Daten zu empfangen. In dieser Ausführungsform ist es somit möglich, Daten auf das analytische Messgerät zu übertragen, ohne dass dessen Spannungsversorgung aktiv ist. So können auf diese Weise z.B. Daten ohne Einlegen einer Batterie oder eines Akkus auf das Messgerät übertragen werden. Dies ist vorteilhaft, wenn beispielsweise länderspezifische Daten, wie Spracheinstellungen, Menüs zur Bedienung, Datum, Uhrzeit, etc. auf ein bereits verpacktes Messgerät übertragen werden sollen. Hier ist eine Einstellung (von Datum, Uhrzeit, etc.) im Rahmen des werksseitigen Systems möglich. Alternativ können solche Einstellungen als Service im Rahmen des werksfernen Systems geleistet werden.

Hierbei umfasst die erste Datenempfangsvorrichtung bevorzugt ein Transponder-Lesemodul zum Auslesen von in einem Transponder mit RFID-Tag (wie zuvor beschrieben) gespeicherten Daten, wobei das Transponder-Lesemodul eingerichtet ist, um ohne eigene Spannungsquelle drahtlos Daten von einer externen Datenübertragungsvorrichtung, insbesondere von einem externen Transponder-Schreibmodul, zu empfangen. Der Ausdruck "externe Datenübertragungsvorrichtung" bedeutet hierbei, dass die Datenübertragungsvorrichtung nicht Bestandteil des tragbaren analytischen Messgerätes ist, sondern außerhalb und unabhängig von diesem angeordnet ist. Das Transponder-Lesemodul des Messgerätes weist üblicherweise einen Schwingkreis auf, der eine spulenförmige Antenne und einen Parallelkondensator umfasst. An diesen Schwingkreis sind ein Hochfrequenz(HF)-Generator, ein Modulator, ein Entkoppelfilter und ein Demodulator geschaltet. Diese Schaltungsteile sind nur unter Betriebsspannung aktiv. Vorzugsweise liefert in diesem Fall die erste Datenempfangsvorrichtung bzw. das Transponder-Lesemodul die Energie für den Transponder. Auf diese Weise können Daten aus dem Transponder ausgelesen werden, wenn eine Betriebsspannung bereitgestellt wird (um Daten auf den Transponder zu übertragen, kann das Messgerät eine Datenübertragungsvorrichtung, insbesondere ein Transponder-Schreibmodul, aufweisen). Damit der Transponder ohne eigene Spannungsversorgung ausgelesen werden kann, wird parallel zu den vorhandenen Komponenten der ersten Datenempfangsvorrichtung eine passive Transponderstruktur geschaltet. Hierzu wird mindestens eine Diode und ein Speicherkondensator zugeschaltet. In der Regel werden zusätzliche Komponenten wie ein Speicherelement, z.B. ein EEPROM, eine Steuerschaltung und ein Lastmodulator eingesetzt. Diese Komponenten sind kommerziell erhältlich, z.B. in Form fertig montierter Chips. Ein Beispiel für einen besonders geeigneten Transponder-Baustein, bei dem ein EEPROM-Speicherelement sowohl über eine Drahtschnittstelle als auch über eine Radiofrequenz-Schnittstelle angesprochen werden kann, ist AT24RF08C der Firma ATMEL.

Bei dieser Ausführungsform spricht das Transponder-Lesemodul der ersten Datenempfangsvorrichtung unter Betriebsspannung nicht nur in der Nähe befindliche Transponder, z.B. einen einer Verpackungseinheit für Verbrauchsmittel zugeordneten Transponder, an, sondern auch den passiven Transponder des tragbaren analytischen Messgerätes selbst. Da heutzutage jeder Transponder über eine individuelle Identifizierung, insbesondere eine eindeutige Identifikationsnummer, verfügt, kann jedoch sichergestellt werden, dass jeweils nur auf den gewünschten Transponder zugegriffen wird. Alternativ oder zusätzlich kann auch ein Authentifizierungselement (analog dem zuvor beschriebenen Authentifizierungselement) vorgesehen werden, durch welches der Zugriff auf den Transponder kontrolliert, d.h. authorisiert oder verweigert, wird.

Ein weiterer Gegenstand der vorliegenden Erfindung ist ein Analysesystem mit erweiterter Benutzerinformation, umfassend
- ein tragbares analytisches Messgerät zur Messung von Stoffkonzentrationen in fluiden Medien, das eine erste Datenempfangsvorrichtung zum Auslesen von in einem beschreibbaren nichtflüchtigen Datenspeicher gespeicherten Daten und ein Anzeigeelement zum Anzeigen von Messergebnissen aufweist;
   wobei die erste Datenempfangsvorrichtung eingerichtet ist, um ohne eigene Spannungsquelle drahtlos Daten zu empfangen. Das erfindungsgemäße Analysesystem kann weiterhin insbesondere umfassen:
- eine Verpackungseinheit für Verbrauchsmittel, die zur Messung von Stoffkonzentrationen in fluiden Medien und/oder zur Vorbereitung einer solchen Messung verwendet werden, wobei der beschreibbare nichtflüchtige Datenspeicher der Verpackungseinheit zugeordnet ist;
- eine erste Datenübertragungsvorrichtung zum Übertragen von Steuerungsdaten auf den beschreibbaren nichtflüchtigen Datenspeicher; und/oder
- eine zweite Datenübertragungsvorrichtung zum Übertragen von Anzeigedaten auf den beschreibbaren nichtflüchtigen Datenspeicher.

Das Anzeigeelement des tragbaren analytischen Messgerätes ist vorzugsweise zum Anzeigen mindestens eines Teils der Anzeigedaten eingerichtet. In Bezug auf weitere Bestandteile und Ausgestaltungen dieses Erfindungsgegenstands wird auf die vorhergehenden Teile der Beschreibung, insbesondere auf die bisher genannten Ausführungsformen, Bezug genommen. Vorzugsweise ist hierbei die erste Datenempfangsvorrichtung ausgestaltet, wie zuvor für den Fall des drahtlosen Datenempfangs ohne eigene Spannungsquelle beschrieben.

Ein weiterer Gegenstand der vorliegenden Erfindung ist ein Analyseverfahren mit erweiterter Benutzerinformation, umfassend folgende Schritte:
(i) Bereitstellen von Verbrauchsmitteln, die von tragbaren analytischen Messgeräten zur Messung von Stoffkonzentrationen in fluiden Medien und/oder zur Vorbereitung einer solchen Messung verwendet werden, und von einer Verpackungseinheit für diese Verbrauchsmittel;
(ii) Übertragen von Steuerungsdaten auf einen beschreibbaren nichtflüchtigen Datenspeicher mittels einer ersten Datenübertragungsvorrichtung, und Zuordnung des beschreibbaren nichtflüchtigen Datenspeichers zu der Verpackungseinheit;
(iii) Übertragen von Anzeigedaten auf den beschreibbaren nichtflüchtigen Datenspeicher mittels einer zweiten Datenübertragungsvorrichtung;
(iv) Auslesen der Steuerungsdaten und der Anzeigedaten aus dem beschreibbaren nichtflüchtigen Datenspeicher mittels einer ersten Datenempfangsvorrichtung, die Bestandteil des tragbaren analytischen Messgerätes ist;
(v) Anzeigen zumindest eines Teils der Anzeigedaten auf einer Anzeigevorrichtung, die Bestandteil des tragbaren analytischen Messgerätes ist; und
(vi) Verwendung der Steuerungsdaten zur Durchführung der analytischen Funktionen des tragbaren analytischen Messgerätes.

Zur Durchführung des erfindungsgemäßen Analyseverfahrens eignet sich insbesondere ein erfindungsgemäßes Analysesystem, wie zuvor beschrieben. So können speziell die zuvor beschriebenen tragbaren analytischen Messgeräte, Verbrauchsmittel und Verpackungseinheiten eingesetzt werden. Weiterhin können insbesondere die zuvor beschriebenen Datenübertragungs- und -empfangsvorrichtungen bzw. beschreibbaren Datenspeicher eingesetzt werden. Die Begriffe Steuerungsdaten und Anzeigedaten sind wie zuvor beschrieben zu verstehen. Vorzugsweise ist das Anzeigeelement des tragbaren analytischen Messgerätes zum Anzeigen mindestens eines Teils der Anzeigedaten eingerichtet.

In einer bevorzugten Ausführungsform der Erfindung wird in Schritt (i) als Verpackungseinheit für die Verbrauchsmittel ein Behältnis zur magazinierten Aufbewahrung mehrerer der Verbrauchsmittel bereitgestellt, wobei die Behältnisse insbesondere ausgewählt sind unter Trommel-Magazinen, Stangen-Magazinen, Reihen-Magazinen und Bandkassetten.

Die Ermittlung chargenspezifischer Kenndaten (Steuerungsdaten) erfolgt üblicherweise so, wie zuvor beschrieben. Die chargenspezifischen Steuerungsdaten werden gemäß Schritt (ii) mittels der ersten Datenübertragungsvorrichtung auf den beschreibbaren nichtflüchtigen Datenspeicher übertragen, wobei der Datenspeicher vor oder nach dem Übertragen der chargenspezifischen Steuerungsdaten der Verpackungseinheit zugeordnet wird. Das Übertragen von Steuerungsdaten auf den beschreibbaren Datenspeicher mittels der ersten Datenübertragungsvorrichtung gemäß Schritt (ii) kann drahtlos oder über einen elektrischen Kontakt erfolgen. Bevorzugt überträgt die erste Datenübertragungsvorrichtung in Schritt (ii) die Steuerungsdaten auf einen ersten Speicherbereich des beschreibbaren nichtflüchtigen Datenspeichers, der nur einmal mit Daten beschreibbar ist.

Die Zuordnung des beschreibbaren nichtflüchtigen Datenspeichers zu der Verpackungseinheit gemäß Schritt (ii) kann derart erfolgen, dass der beschreibbare Datenspeicher fest mit der Verpackungseinheit verbunden angeordnet ist. Dies kann z. B. realisiert werden, indem der Datenspeicher in die Verpackungseinheit integriert wird, beispielsweise in ein (Verpackungs-)Material wie Kunststoff eingebettet oder von diesem umschlossen, oder indem der beschreibbare Datenspeicher auf einer Fläche der Verpackungseinheit, beispielsweise der Oberfläche, fixiert wird, insbesondere durch Verkleben oder Verschweißen. Eine solche Zuordnung des beschreibbaren nichtflüchtigen Datenspeichers zu der Verpackungseinheit in Form einer festen Verbindung ermöglicht insbesondere ein Übertragen der Steuerungsdaten auf den beschreibbaren Datenspeicher, nachdem die Verpackungseinheit zum Verpacken der Verbrauchsmittel eingesetzt wurde. In diesem Fall, dass der Datenspeicher fest mit der Verpackungseinheit verbunden angeordnet ist, wird als beschreibbarer nichtflüchtiger Datenspeicher vorteilhaft ein Transponder, wie zuvor beschrieben, eingesetzt, insbesondere ein Transponder, der ein RFID-Tag aufweist. Erfolgt das Übertragen der Steuerungsdaten auf den beschreibbaren nichtflüchtigen Datenspeicher vor dessen Zuordnung zu der Verpackungseinheit, so kann der beschreibbare Datenspeicher der Verpackungseinheit so beigefügt werden, dass er aus dieser auf einfache Weise entnehmbar ist. In diesem Fall kann der Datenspeicher beispielsweise lose in ein Behältnis für Verbrauchsmittel, z.B. ein Verbrauchsmittel-Magazin, etwa ein Teststreifenröhrchen, eingelegt werden. In diesem Fall kann als beschreibbarer Datenspeicher vorteilhaft z. B. ein EPROM/Flash-Baustein, ein Transponder, und/oder ein ROM-Baustein eingesetzt werden. Es versteht sich, dass der Datenspeicher zusammen mit einem oder mehreren Behältnissen zur Aufbewahrung der Verbrauchsmittel in einer Umverpackung enthalten sein kann.

In einer bevorzugten Ausführungsform erfolgt Schritt (ii) in einem werksseitigen System, wie zuvor definiert.

Das Übertragen der Anzeigedaten auf den beschreibbaren Datenspeicher mittels der zweiten Datenübertragungsvorrichtung gemäß Schritt (iii) kann drahtlos oder über einen elektrischen Kontakt, bevorzugt drahtlos erfolgen. Schritt (iii) erfolgt insbesondere derart, dass
(iii.1) der beschreibbare nichtflüchtige Datenspeicher, gegebenenfalls einschließlich der zugeordneten Verpackungseinheit, in räumliche Nähe zu und/oder in elektrischen Kontakt mit der zweiten Datenübertragungsvorrichtung gebracht wird; und
(iii.2) die zweite Datenübertragungsvorrichtung die Anzeigedaten drahtlos und/oder gegebenenfalls über den elektrischen Kontakt auf den beschreibbaren nichtflüchtigen Datenspeicher überträgt.

Die Herstellung der räumlichen Nähe zwischen dem Datenspeicher und der zweiten Datenübertragungsvorrichtung in Schritt (iii.1) dient dazu, ein drahtloses Übertragen von Daten auf den beschreibbaren Datenspeicher zu ermöglichen. Als ausreichende räumliche Nähe hierfür wird im Allgemeinen eine Distanz im Bereich von 0,01 cm bis 5 m, insbesondere von 0,1 cm bis 1 m in Betracht kommen.

Es ist bevorzugt, dass die Anzeigedaten drahtlos übertragen werden. Hierdurch kann auf einen elektrischen Kontakt zur Datenübertragung verzichtet werden. Dementsprechend erfolgt Schritt (iii.1) vorzugsweise derart, dass der beschreibbare nichtflüchtige Datenspeicher einschließlich der zugeordneten Verpackungseinheit in räumliche Nähe zu der zweiten Datenübertragungsvorrichtung gebracht wird; und Schritt (iii.2) erfolgt vorzugsweise derart, dass die zweite Datenübertragungsvorrichtung die Anzeigedaten drahtlos auf den beschreibbaren nichtflüchtigen Datenspeicher überträgt.

Vorzugsweise überträgt in Schritt (iii) die zweite Datenübertragungsvorrichtung die Anzeigedaten auf einen zweiten Speicherbereich des beschreibbaren nichtflüchtigen Datenspeichers, der mindestens einmal und vorzugsweise mehrfach mit Daten beschreibbar ist. Besonders bevorzugt erfolgt die Übertragung der Anzeigedaten in Schritt (iii) erst nach erfolgreicher Authentifizierung durch ein Authentifizierungselement des beschreibbaren nichtflüchtigen Datenspeichers, durch welches ein Auslesen und/oder Beschreiben des zweiten Speicherbereichs des beschreibbaren nichtflüchtigen Datenspeichers autorisiert wird. Die hierfür anwendbaren Vorgehensweisen sind dem Fachmann grundsätzlich bekannt und wurden weiter oben bereits erläutert.

In einer bevorzugten Ausführungsform erfolgt Schritt (iii) in einer Vertriebsstelle und/oder Distributionsstelle, besonders bevorzugt in einer Distributionsstelle, eines werksfernen Systems, wie zuvor definiert.

Das Übertragen der Steuerungsdaten auf den beschreibbaren nichtflüchtigen Datenspeicher mittels der ersten Datenempfangsvorrichtung des tragbaren analytischen Messgerätes gemäß Schritt (iv) kann drahtlos oder über einen elektrischen Kontakt, bevorzugt drahtlos erfolgen. Schritt (iv) erfolgt insbesondere derart, dass
(iv.1) der beschreibbare nichtflüchtige Datenspeicher, gegebenenfalls einschließlich der zugeordneten Verpackungseinheit, in räumliche Nähe zu und/oder in elektrischen Kontakt mit der ersten Datenempfangsvorrichtung des tragbaren analytischen Messgerätes gebracht wird; und
(iv.2) die erste Datenempfangsvorrichtung die Steuerungsdaten und Anzeigedaten drahtlos und/oder gegebenenfalls über den elektrischen Kontakt aus dem beschreibbaren nichtflüchtigen Datenspeicher ausliest.

Die Herstellung der räumlichen Nähe zwischen dem Datenspeicher und der zweiten Datenübertragungsvorrichtung in Schritt (iv.1) dient dazu, ein drahtloses Empfangen von Daten aus dem Datenspeicher zu ermöglichen. Als ausreichende räumliche Nähe hierfür wird im Allgemeinen eine Distanz im Bereich von 0,01 cm bis 50 cm, insbesondere von 0,05 cm bis 5 cm in Betracht kommen.

Es ist bevorzugt, dass die Anzeigedaten drahtlos ausgelesen werden. Hierdurch kann auf einen elektrischen Kontakt zur Datenübertragung verzichtet werden. Dementsprechend erfolgt Schritt (iv.1) vorzugsweise derart, dass der beschreibbare nichtflüchtige Datenspeicher einschließlich der zugeordneten Verpackungseinheit in räumliche Nähe zu der ersten Datenempfangsvorrichtung des tragbaren analytischen Messgerätes gebracht wird; und Schritt (iv.1) erfolgt vorzugsweise derart, dass die erste Datenempfangsvorrichtung die Steuerungsdaten und Anzeigedaten drahtlos aus dem beschreibbaren nichtflüchtigen Datenspeicher ausliest.

Die räumliche Nähe zwischen dem Datenspeicher und der zweiten Datenübertragungsvorrichtung in Schritt (iv.1) kann insbesondere erzielt werden, indem die Verpackungseinheit mit dem Datenspeicher oder der Datenspeicher selbst einer hierfür vorgesehenen Positionierungshilfe, wie zuvor beschrieben, an der Oberfläche des Gehäuses des tragbaren analytischen Messgerätes zugeführt wird. Alternativ kann diese räumliche Nähe erzielt werden, indem die Verpackungseinheit mit dem Datenspeicher oder der Datenspeicher selbst einem hierfür vorgesehenen Aufnahmeelement, wie zuvor beschrieben, des tragbaren analytischen Messgerätes zugeführt wird. Wenn der beschreibbare nichtflüchtige Datenspeicher selbst (und nicht die zugeordnete Verpackungseinheit) der Positionierungshilfe bzw. dem Aufnahmeelement zugeführt wird, so wird der Datenspeicher der Verpackungseinheit entnommen, bevor er der Positionierungshilfe bzw. dem Aufnahmeelement zugeführt wird. Vorzugsweise wird die räumliche Nähe zwischen dem Datenspeicher und der zweiten Datenübertragungsvorrichtung in Schritt (iv.1) dadurch erzielt, dass die Verpackungseinheit einschließlich des zugeordneten beschreibbaren nichtflüchtigen Datenspeichers
(iv.1a) einem hierfür vorgesehenen Aufnahmeelement des tragbaren analytischen Messgerätes zugeführt wird; oder
(iv.1b) einer hierfür vorgesehenen Positionierungshilfe an der Oberfläche des Gehäuses des tragbaren analytischen Messgerätes zugeführt wird.

Besonders bevorzugt veranlasst die Zuführung der Verpackungseinheit zu dem Aufnahmeelement gemäß Schritt (iv.1 a) oder zu der Positionierungshilfe gemäß Schritt (iv.1b) die erste Datenempfangsvorrichtung dazu, gemäß Schritt (iv.2) die Steuerungsdaten und Anzeigedaten drahtlos aus dem beschreibbaren nichtflüchtigen Datenspeicher auszulesen. Dies kann insbesondere realisiert werden, indem die erste Datenempfangsvorrichtung wiederholt in zeitlichen Abständen ein Signal aussendet, welches den in der Nähe befindlichen Datenspeicher, der speziell einen Transponder mit RFID-Tag umfasst, zum Senden der Steuerungsdaten und Anzeigedaten anregt. Alternativ kann der Datenspeicher, der speziell einen Transponder mit RFID-Tag umfasst, durch das Schließen einer Klappe im Gehäuse des Messgerätes zum Senden der Steuerungsdaten und Anzeigedaten angeregt werden. Dieser letztere Fall bietet sich vor Allem an, wenn Verbrauchsmittel-Magazine verwendet werden, die vollständig in das Messgerät eingeführt werden. Die in diesem Zusammenhang erforderlichen Schaltungstechniken sind dem Fachmann bekannt und beispielsweise in Finkenzeller, Klaus, RFID-Handbuch, 2. Aufl., Carl Hanser Verlag, München 2000 beschrieben.

Wenn die Anzeigedaten in Schritt (iv) über einen elektrischen Kontakt ausgelesen werden sollen, so erfolgt Schritt (iv.1) insbesondere derart, dass der beschreibbare nichtflüchtige Datenspeicher der Verpackungseinheit entnommen wird und in elektrischen Kontakt mit der ersten Datenempfangsvorrichtung des tragbaren analytischen Messgerätes gebracht wird. Hierbei wird der der Verpackungseinheit entnommene Datenspeicher vorzugsweise einem hierfür vorgesehenen Aufnahmeelement des Messgerätes zugeführt. Schritt (iv.2) erfolgt hierbei insbesondere derart, dass die erste Datenempfangsvorrichtung die Steuerungsdaten und Anzeigedaten über den elektrischen Kontakt aus dem beschreibbaren nichtflüchtigen Datenspeicher ausliest. Vorteilhaft ist hierbei der Transponder selbst so ausgestaltet, dass er über einen elektrischen Kontakt mittels der ersten Datenempfangsvorrichtung des tragbaren analytischen Messgerätes auslesbar ist. Dies kann z.B. in einem (drahtlos ansprechbaren) Transponder realisiert werden, der einerseits mit einem elektronischen Speicherelement ausgestattet ist und andererseits elektrische Kontakte aufweist. Hierfür geeignete sogenannte Dual-Port-EEPROM-Speicherelemente (z.B. von der Firma ATMEL (ATMEL AT24RF08C)) sind weiter oben bereits beschrieben. Somit ist hier eine Ausgestaltung des tragbaren analytischen Messgerätes möglich, ohne dass zusätzlich zu einer elektrischen Kupplung am Messgerät ein weiteres Lesemodul zum Auslesen der auf dem Transponder gespeicherten Daten (Anzeigedaten) vorgesehen werden müsste.

Das erfindungsgemäße Analyseverfahren zeichnet sich dadurch aus, dass die Schritte (v) und/oder (vi) unabhängig voneinander und/oder mehrfach wiederholt ausgeführt werden können. Hierbei kommt es in der Regel nicht auf eine zeitliche Reihenfolge der beiden Schritte an, so dass Schritt (vi) gegebenenfalls vor Schritt (v) durchgeführt werden kann. In einer Ausführungsform der Erfindung kann jedoch vorgesehen werden, dass Schritt (vi) erst nach Durchführung von Schritt (v) vorgenommen werden kann, beispielsweise indem der Anwender die Kenntnisnahme der auf dem Anzeigeelement des Messgerätes angezeigten Anzeigedaten bestätigen muss, etwa durch Betätigen eines Bedienknopfes.

Noch eine bevorzugte Ausführungsform der Erfindung umfasst weiterhin folgende Schritte
(vii) mindestens eine Messung einer Analytkonzentration in Blut durch das analytische Messgerät, wobei man mindestens ein Messergebnis und/oder die Messung betreffende Messdaten erhält;
(viii) mindestens teilweises Übertragen des Messergebnisses und/oder der Messdaten auf den beschreibbaren Datenspeicher mittels einer dritten Datenübertragungsvorrichtung, die Bestandteil des analytischen Messgerätes ist, insbesondere auf automatische Weise; und
(ix) Auslesen des Messergebnisses und/oder der Messdaten aus dem beschreibbaren Datenspeicher, insbesondere auf automatische Weise, mittels
   (ix.1) einer zweiten Datenempfangsvorrichtung, die Bestandteil eines werksfernen Systems, wie zuvor definiert, ist; oder
   (ix.2) einer dritten Datenempfangsvorrichtung, die Bestandteil eines werksseitigen Systems, wie zuvor definiert, ist; und
(x) gegebenenfalls Übermittlung mindestens eines Teils des Messergebnisses und/oder der Messdaten, das/die in Schritt (ix.1) ausgelesen wurde/wurden, an die dritte Datenempfangsvorrichtung, die Bestandteil eines werksseitigen Systems, wie zuvor definiert, ist.

Die hier beschriebene Ausführungsform ermöglicht es, dass zumindest ein Teil des Messergebnisses und/oder der Messdaten des Anwenders für eine Auswertung zur Verfügung gestellt werden können. Eine solche Auswertung kann zur Unterstützung einer Behandlung oder Therapie, z.B. für einen Arzt, Apotheker oder eine Krankenschwester, von Interesse sein, aber auch für den Hersteller der Verbrauchsmittel, insbesondere im Zusammenhang mit bestimmten Gerätezuständen, z.B. Fehlermeldungen und Systemzustände wie Batterie oder Verstärkungseinstellung. Für eine solche Auswertung ist es erforderlich, dass die Messergebnisse und/oder Messdaten, die auf dem beschreibbaren Datenspeicher enthalten sind, zumindest teilweise an eine hierfür vorgesehene Datenempfangsvorrichtung übermittelt werden. Diese Übermittlung kann auf verschiedene Weisen bewirkt werden.

Erstens ist es möglich, dass der Anwender den beschreibbaren Datenspeicher selbst, der zumindest einen Teil des Messergebnisses und/oder der Messdaten des Anwenders enthält, zurückgeben kann. Diese Rückgabe erfolgt vorteilhaft zusammen mit der Rückgabe benutzter Verbrauchsmittel bzw. Verpackungsmaterialien, die der Anwender zur Entsorgung zurückgibt. Als Annahmestellen für benutzte Verbrauchsmittel bzw. Verpackungsmaterialien zusammen mit dem beschreibbaren Datenspeicher sind insbesondere geeignet: (i) Vertriebsstellen zum Handel mit den Verpackungseinheiten, wie zuvor definiert, und (ii) Distributionsstellen zur Distribution der Verpackungseinheiten an einen Endverbraucher, wie zuvor definiert, mithin insbesondere ein Arzt, Apotheker oder eine Krankenschwester bzw. eine Arztpraxis, Apothekenniederlassung oder Krankenhausabteilung. Somit ist eine Annahmestelle in diesem Sinne in der Regel ein Bestandteil eines werksfernen Systems, wie zuvor definiert. In diesem Fall erfolgt das Auslesen des Messergebnisses und/oder der Messdaten aus dem beschreibbaren Datenspeicher gemäß Schritt (ix.1)mittels einer zweiten Datenempfangsvorrichtung, die Bestandteil eines werksfernen Systems, wie zuvor definiert, ist. Ferner erfolgt hierbei vorzugsweise eine Übermittlung mindestens eines Teils des Messergebnisses und/oder der Messdaten, das/die in Schritt (ix.1) ausgelesen wurde/wurden, gemäß Schritt (x) an die dritte Datenempfangsvorrichtung des werksseitigen Systems, wie zuvor definiert. Der Anwender kann seine benutzten Verbrauchsmittel, Verpackungsmaterialien bzw. Datenspeicher auch auf dem Postweg an eine Annahmestelle zurückgeben. In diesem Fall kann die Annahmestelle entweder Bestandteil eines werksfernen Systems oder eines werksseitigen Systems, jeweils wie zuvor definiert, sein. Ist die Annahmestelle Bestandteil eines werksseitigen Systems, wie zuvor definiert, so erfolgt das Auslesen des Messergebnisses und/oder der Messdaten aus dem beschreibbaren Datenspeicher gemäß Schritt (ix.2) mittels der dritten Datenempfangsvorrichtung, die Bestandteil eines werksseitigen Systems, wie zuvor definiert, ist.

Zweitens ist es möglich, dass zumindest ein Teil des Messergebnisses und/oder der Messdaten des Anwenders, das/die auf dem beschreibbaren Datenspeicher enthalten ist/sind, gemäß Schritt (ix.2) von einer dritten Datenempfangsvorrichtung, die Bestandteil eines werksseitigen Systems, wie zuvor definiert, ist, derart ausgelesen wird, dass die Übermittlung der auf dem beschreibbaren Datenspeicher enthaltenen Daten mittels Telekommunikationseinrichtungen erfolgt. Geeignete Telekommunikationseinrichtungen sind z.B. Internet, Telefon, Mobiltelefon.

Für die vorliegende Erfindung wird vorteilhaft dafür Sorge getragen, dass die verschiedenen Daten (Steuerungsdaten, Anzeigedaten, Messergebnisse und/oder Messdaten) jeweils nur von hierzu autorisierten Nutzern bzw. Anwendern auf den beschreibbaren Datenspeicher geschrieben und/oder aus diesem ausgelesen werden können. Zu diesem Zweck werden den unterschiedlichen Nutzern bzw. Anwendern für den jeweiligen Zweck angepasste Rechte zugeordnet. So können z.B. nur Leserechte oder nur Schreibrechte vergeben werden. Auch Schreibrechte nur auf einen freigegebenen Bereich des beschreibbaren Datenspeichers sind möglich. Beschreibbare Datenspeicher, die verschiedene entsprechende Speicherbereiche (z.B. nur einmal beschreibbar, mehrfach beschreibbar, geschützt oder frei beschreibbar) aufweisen, sind bekannt und kommerziell verfügbar. Verschlüsselungs- und Authentifikationsverfahren, um die Zugriffsrechte auf die verschiedenen Speicherbereiche zu verwalten, sind dem Fachmann ebenfalls bekannt, z.B. das sogenannte Ticketing (RFID-Technik im eTicketing, H. Kelter et al., 11. Deutscher IT-Sicherheitskongress, Bundesamt für Sicherheit in der Informationstechnik, 13.05.2009). Beispiele für unterschiedliche Zugriffsrechte sind insbesondere:
- Der Endverbraucher erhält Leserechte nur für die Anzeigedaten, insbesondere nur für den zweiten Speicherbereich des beschreibbaren Datenspeichers, und gegebenenfalls für einen frei beschreibbaren dritten Speicherbereich des beschreibbaren Datenspeichers.
- Der Endverbraucher erhält Schreibrechte nur für einen frei beschreibbaren dritten Speicherbereich des beschreibbaren Datenspeichers, falls vorhanden.
- Der Endverbraucher kann den Umfang der Daten, die an das werksferne und/oder werksseitige System zurückgegeben werden, selbst definieren, z.B. über Softwarefunktionen des analytischen Messgerätes; so kann dem berechtigten Interesse nach Datenschutz entsprochen werden.
- Der Hersteller der Verbrauchsmittel bzw. Verpackungseinheiten erhält volle Schreib- und Leserechte für alle Speicherbereiche des beschreibbaren Datenspeichers, insbesondere für den ersten Speicherbereich des beschreibbaren Datenspeichers.
- Vertriebsstellen und/oder Distributionsstellen, jeweils wie zuvor definiert, erhalten Schreibrechte für die Anzeigedaten, insbesondere für den zweiten Speicherbereich des beschreibbaren Datenspeichers, und gegebenenfalls für einen frei beschreibbaren dritten Speicherbereich des beschreibbaren Datenspeichers. Die Vergabe von Rechten kann über eine spezifische Software erfolgen, beispielsweise über Internet, Telefon oder Datenträger.

Im Folgenden wird die Erfindung anhand von Beispielen unter Verwendung der beigefügten Figuren näher erläutert. Die Beispiele dienen allein zur Veranschaulichung einzelner allgemeiner und/oder spezieller Aspekte der Erfindung, sie sind jedoch in keiner Weise als einschränkend zu verstehen.

### Beschreibung der Figuren

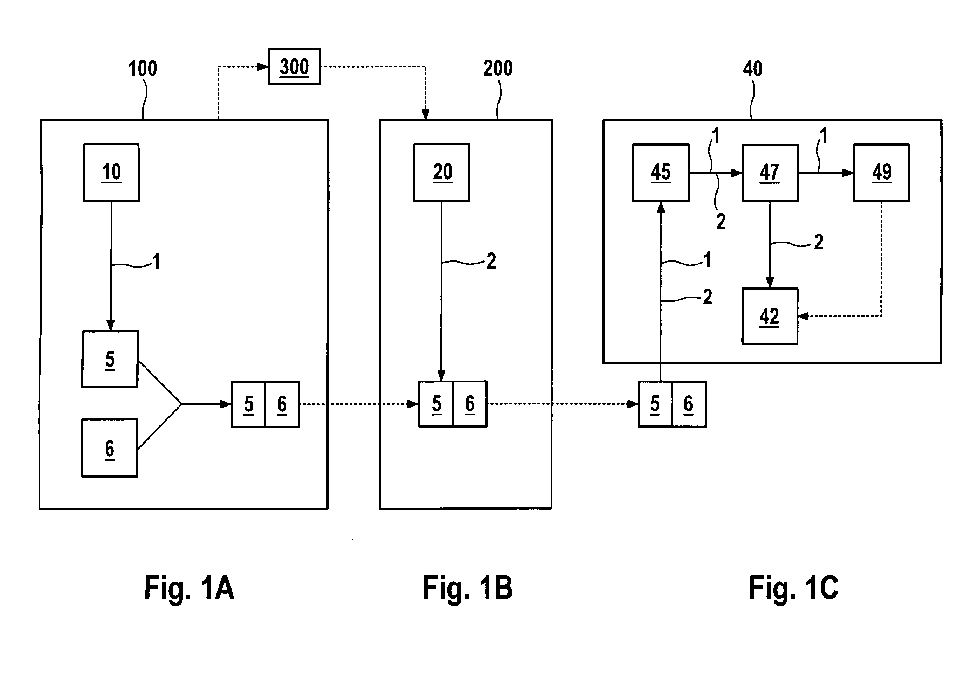

Fig. 1 stellt schematisch eine bevorzugte Ausführungsform des erfindungsgemäßen Analysesystems dar.

### Bezugszeichenliste:

- 1: Steuerungsdaten
- 2: Anzeigedaten
- 5: beschreibbarer, nichtflüchtiger Datenspeicher
- 6: Verpackungseinheit für Verbrauchsmittel
- 7, 8, 9: Datenübertragung
- 10: erste Datenübertragungsvorrichtung
- 20: zweite Datenübertragungsvorrichtung
- 40: tragbares analytisches Messgerät
- 42: Anzeigeelement zum Anzeigen von Messergebnissen
- 45: erste Datenempfangsvorrichtung
- 47: Kontrolleinheit
- 49: Steuerungsvorrichtung zur Steuerung einer Messung
- 100: werksseitiges System
- 200: werksfernes System
- 300: Übertragungsvorrichtung für Daten und/oder Rechte
- 400: Materialrücklauf(Verpackungseinheit, Verbrauchsmittel und/oder Datenspeicher)
- 500: Verpackung des analytischen Messegrätes

### Beispiel 1

Es wird auf Fig. 1 Bezug genommen, welche eine schematische Übersicht über ein bevorzugtes erfindungsgemäßes Analysesystem darstellt.

Fig. 1A zeigt beispielhaft ein werksseitiges System (100) im Sinne der vorliegenden Erfindung. Das werksseitige System (100) weist zumindest eine erste Datenübertragungsvorrichtung (10) zum Übertragen von Steuerungsdaten (1) auf einen beschreibbaren Datenspeicher (5) auf. Der beschreibbare Datenspeicher (5) umfasst vorzugsweise einen RFID-Tag, sodass das Speichern und Auslesen von Daten drahtlos erfolgen kann. Der beschreibbare Datenspeicher (5) weist mindestens einen ersten und einen zweiten Speicherbereich und gegebenenfalls noch einen dritten Speicherbereich auf, wobei der erste Speicherbereich nur einmal beschreibbar ist und der zweite und dritte Speicherbereich jeweils mehrfach beschreibbar sind. Die Steuerungsdaten (1) werden mittels der ersten Datenübertragungsvorrichtung (10) in den ersten Speicherbereich des beschreibbaren Datenspeichers (5) übertragen. Der die Steuerungsdaten (1) enthaltende Datenspeicher (5) wird zusammen mit mindestens einem Teil der Charge der Verbrauchsmittel, für welche die Steuerungsdaten (1) erstellt wurden, in die Verpackungseinheit (6), z. B. ein zylindrisches Kunststoff-oder Metallröhrchen, gegeben. Im Fall von Teststreifen zur Bestimmung des Glukosegehaltes in Blut als Verbrauchsmaterial werden üblicherweise 50 Teststreifen in eine Einheit des Verpackungsmaterials (6), z. B. ein zylindrisches Kunststoff oder Metallröhrchen, gegeben.

Die Erstellung der Steuerungsdaten (1, hier chargenspezifische Kenndaten), erfolgt hierbei so, dass eine Charge der Verbrauchsmaterialien hergestellt wird, von dieser Charge eine Teilmenge entnommen wird, und anhand der entnommenen Teilmenge die chargenspezifischen Steuerungsdaten (1) gewonnen werden. Diese chargenspezifischen Steuerungsdaten (1) werden erfindungsgemäß mittels der ersten Datenübertragungsvorrichtung (10) auf den beschreibbaren Datenspeicher (5) übertragen.

Optional kann die erste Datenübertragungsvorrichtung (10) auch genutzt werden, um in dem werksseitigen System (100) auch Anzeigedaten und/oder weitere Daten auf den beschreibbaren Datenspeicher (5) zu übertragen, sofern dies gewünscht wird.

In dem werksseitigen System (100) wird der beschreibbare Datenspeicher (5) der Verpackungseinheit (6) zugeordnet. Dies kann erfolgen, indem der beschreibbare nichtflüchtige Datenspeicher (5) fest mit der Verpackungseinheit (6) verbunden angeordnet wird. In diesem Fall weist das tragbare analytische Messgerät (40, siehe Fig. 1C) vorteilhaft ein Aufnahmeelement zur Aufnahme der Verpackungseinheit (6) und/oder eine Positionierungshilfe zur Positionierung der Verpackungseinheit (6) relativ zum analytischen Messgerät auf. Alternativ kann die Zuordnung des beschreibbaren Datenspeichers (5) zu der Verpackungseinheit (6) derart erfolgen, dass der Datenspeicher (5) der Verpackungseinheit (6) entnehmbar beigefügt wird. In diesem Fall weist das tragbare analytische Messgerät vorteilhaft ein Aufnahmeelement zur Aufnahme des beschreibbaren nichtflüchtigen Datenspeichers (5) auf. Die Verpackungseinheit (6) mit dem zugeordneten beschreibbaren Datenspeicher (5) kann über eine Vertriebsstelle zum Handel mit den Verpackungseinheiten (6) oder eine Distributionsstelle zur Distribution der Verpackungseinheiten (6) an einen Endverbraucher weiter vertrieben werden.

Fig. 1B stellt schematisch ein werksfernes System (200) im Sinne der vorliegenden Erfindung dar. Das werksferne System (200) umfasst insbesondere eine Vertriebsstelle zum Handel mit den Verpackungseinheiten (6) oder eine Distributionsstelle zur Distribution der Verpackungseinheiten (6) an einen Endverbraucher. Das werksferne System (200) bzw. die Vertriebsstelle und/oder Distributionsstelle weist eine zweite Datenübertragungsvorrichtung (20) zum Übertragen von Anzeigedaten (2) auf den beschreibbaren Datenspeicher (5) auf. Die zweite Datenübertragungsvorrichtung (20) umfasst vorzugsweise ein Transponder-Schreibmodul zum Übertragen von Anzeigedaten (2), sodass die Übertragung von Daten drahtlos erfolgen kann. Über die optionale Übertragungsvorrichtung (300) können Daten und/oder Rechte, z.B. Schreib- und/oder Leserechte, von dem werksseitigen System an das werksferne System übertragen werden, insbesondere mittels Internet, Telefon und/oder Datenträger.

Die zweite Datenübertragungsvorrichtung (20) kann ein Distanzerkennungsmodul umfassen. Das Distanzerkennungsmodul umfasst ein Sendeelement, das in regelmäßigen Abständen von etwa 1 bis 60 Sekunden drahtlos ein Signal aussendet, z.B. mit einer Frequenz von etwa 900 MHz. Das Signal dient dazu, den RFID-Tag des beschreibbaren Datenspeichers (5) zum drahtlosen Senden eines Antwortsignals anzuregen, welches von einem Empfangselement des Distanzerkennungsmoduls empfangen und erkannt werden kann. Das vom Empfangselement empfangene Antwortsignal wird vom Distanzerkennungsmodul an das Transponder-Schreibmodul weitergeleitet und veranlasst dieses automatisch, die Anzeigedaten (2) in den RFID-Tag des beschreibbaren Datenspeichers (5) zu übertragen. Die zweite Datenübertragungsvorrichtung (20) kann als spezifische Entwicklung zur Verfügung gestellt werden, oder eine Standard-Lesevorrichtung mit spezifischer Software sein, wobei die spezifische Software z.B. über die Übertragungsvorrichtung (300) übertragen werden kann. Eine Synchronisation erfolgt hierbei über die Software, das Messgerät bzw. die zweite Datenübertragungsvorrichtung (20).

Über die Vertriebsstelle zum Handel mit den Verpackungseinheiten (6) und/oder über die Distributionsstelle zur Distribution der Verpackungseinheiten (6) kann die Verpackungseinheit (6) mit dem zugeordneten beschreibbaren Datenspeichers (5) an einen Endverbraucher gelangen.

Fig. 1C stellt schematisch ein tragbares analytisches Messgerät (40) im Sinne der vorliegenden Erfindung dar. Zusätzlich ist die Verpackungseinheit (6) mit dem zugeordneten beschreibbaren Datenspeichers (5) dargestellt. Das tragbare analytische Messgerät (40) zur Messung von Stoffkonzentrationen in fluiden Medien weist das eine erste Datenempfangsvorrichtung (45) zum Auslesen von auf dem beschreibbaren Datenspeicher (5) gespeicherten Daten und ein Anzeigeelement (42) zum Anzeigen von Messergebnissen auf. Das tragbare analytische Messgerät (40) weist vorzugsweise entweder ein Aufnahmeelement zur Aufnahme des Datenspeichers (5) oder der Verpackungseinheit (6) auf, oder eine Positionierungshilfe zur Positionierung der Verpackungseinheit (6) relativ zum analytischen Messgerät (40). Die von der ersten Datenempfangsvorrichtung (45) ausgelesenen Daten (Steuerungsdaten (1) und Anzeigedaten (2)) werden an eine Kontrolleinheit (47) mit eigenem Mikroprozessor und eigenem Speicherbaustein geleitet. Die Kontrolleinheit (47) erkennt die Steuerungsdaten (1) und die Anzeigedaten (2). Die Steuerungsdaten (1) werden von der Kontrolleinheit (47) an die Steuerungseinheit (49) zur Durchführung von Messungen mit dem tragbaren analytischen Messgerät (40) weitergeleitet. Die Anzeigedaten (2) werden von der Kontrolleinheit (47) an das Anzeigeelement (42) geleitet. Es kann vorgesehen werden, dass die Durchführung einer Messung erst ermöglicht wird (das heißt durch die Kontrolleinheit (47) freigegeben wird), wenn der Anwender durch Betätigen eines Bedienknopfes die Kenntnisnahme der auf dem Anzeigeelement (42) angezeigten Anzeigedaten (2) bestätigt hat.

Zur Durchführung einer Messung wird ein Verbrauchsmittel oder eine Verpackungseinheit (6) mit Verbrauchsmitteln, wie Teststreifen oder eine Testbandkassette zur Blutglukosebestimmung, in eine dafür vorgesehene Öffnung des Messgerätes (40) eingeführt. Nach Durchführung der Messung wird das Messergebnis auf dem Anzeigeelement (42) angezeigt.

### Beispiel 2

Es wird auf Fig. 1A bis 1C Bezug genommen. Das in diesem Beispiel beschriebene Analyseverfahren wird mithilfe des in Beispiel 1 beschriebenen Analysesystems durchgeführt, so dass in diesem Zusammenhang auf alle Merkmale der konstruktiven Ausgestaltung des Analysesystems aus Beispiel 1 Bezug genommen wird.

Bei dem Analyseverfahren wird in Schritt (i) eine Verpackungseinheit (6), hier ein zylindrisches Kunststoff-oder Metallröhrchen, für Verbrauchsmittel, z.B. Teststreifen, bereitgestellt. Die Teststreifen (Verbrauchsmittel) werden von dem tragbaren analytischen Messgerät (40, Fig. 1C) zur Messung des Gehaltes von Glucose in Blut verwendet (siehe Schritt (vi)).

In Schritt (ii) werden Steuerungsdaten (1), die wie in Beispiel 1 erstellt wurden, mittels der ersten Datenübertragungsvorrichtung (10) auf den beschreibbaren Datenspeicher (5) übertragen. Die Datenübertragung erfolgt hier, wie in Beispiel 1 erläutert, vorzugsweise drahtlos. Der Datenspeicher (5) wird der Verpackungseinheit (6), hier ein Behältnis in Form eines zylindrischen Röhrchens, zugeordnet, indem er zusammen mit den Verbrauchsmitteln (5) in das Behältnis (6) gegeben wird.

In Schritt (iii) werden mittels der zweiten Datenübertragungsvorrichtung (20) Anzeigedaten (2) auf den beschreibbaren Datenspeicher (5), hier ein RFID-Tag, übertragen. Hierzu werden die in der Verpackungseinheit (6) verpackten Verbrauchsmittel einschließlich des beschreibbaren Datenspeichers (5) in eine solche räumliche Nähe zu der zweiten Datenübertragungsvorrichtung (20) gebracht, dass ein drahtloses Übertragen von Daten auf den beschreibbaren Datenspeicher (5) möglich ist. Diese ausreichende räumliche Nähe kann gegebenenfalls durch ein Distanzerkennungsmodul der zweiten Datenübertragungsvorrichtung (20) automatisch erkannt werden. Die gesamte Datenübertragung kann hier erfolgen, während der Datenspeicher (5) zusammen mit den Verbrauchsmitteln in der Verpackungseinheit (6) verpackt vorliegt. In der europäischen Patentanmeldung mit der Anmeldenummer 09155892.4 sind weitere mögliche Ausgestaltungen eines Distanzerkennungsmoduls beschrieben.

In Schritt (iv) werden die Steuerungsdaten (1) und die Anzeigedaten (2) mittels der Datenempfangsvorrichtung (45) des tragbaren analytischen Messgerätes (40) aus dem beschreibbaren Datenspeicher (5) ausgelesen. Zu diesem Zweck wird der beschreibbare Datenspeicher (5) der Verpackungseinheit (6) entnommen und einem Aufnahmeelement oder einer Positionierungshilfe des Messgerätes (40) zugeführt. Das Auslesen der Steuerungsdaten (1) und der Anzeigedaten (2) erfolgt hier vorteilhaft auf automatische Weise derart, dass das eingeschaltete Messgerät (40) den im Aufnahmeelement (44) befindlichen Datenspeicher (5) erkennt und dadurch die Datenempfangsvorrichtung (45) zum Auslesen der Daten aktiviert. Das Messgerät (40) kann so ausgestaltet sein, dass es durch das Einführen des Datenspeichers (5) in das Aufnahmeelement eingeschaltet wird.

In Schritt (v) wird zumindest ein Teil der Anzeigedaten (2) auf der Anzeigevorrichtung (42) des tragbaren analytischen Messgerätes (40) wiedergegeben.

In Schritt (vi) werden die Steuerungsdaten (1) zur Durchführung der analytischen Funktionen des tragbaren analytischen Messgerätes (40) verwendet. Hierbei betreffen die Steuerungsdaten (1) insbesondere die Umwandlung von Messdaten in Messergebnisse unter Verwendung chargenspezifischer Kenndaten zur Kalibrierung. Die Steuerungsdaten (1) können weitere Funktionen betreffen, die mit der Vorbereitung und Durchführung einer Messung, dem Aufzeichnen und Sammeln von Messdaten und/oder dem Auswerten und Übertragen von Messdaten bzw. Messergebnissen in Zusammenhang stehen. Über die Steuerungseinheit (49) wird die Messung des Gehaltes von Glucose in Blut durch das tragbare analytische Messgerät (40) gesteuert. Zur Durchführung der Messung gibt der Anwender einen Tropfen Blut auf ein dafür vorgesehenes Testfeld auf dem Teststreifen (Verbrauchsmittel) und führt den Teststreifen in die zu dessen Aufnahme vorgesehene Öffnung ein. Die aufgrund der im Testfeld stattfindenden Reaktion zwischen Reagenz-Komponenten und Blutbestandteilen auftretende Farbänderung wird photometrisch oder elektrochemisch detektiert. Mithilfe der Steuerungsdaten (1) wandelt die Steuerungseinheit (49) die Messdaten in ein quantitatives Messergebnis um, welches mittels der Anzeigevorrichtung (42) des tragbaren analytischen Messgerätes wiedergegeben wird.

Über die optionale Übertragungsvorrichtung (300) können Daten, z.B. Messergebnisse oder Messdaten, von dem werksfernen System an das werksseitige System übertragen werden, insbesondere mittels Internet, Telefon und/oder Datenträger.

### Beispiel 3

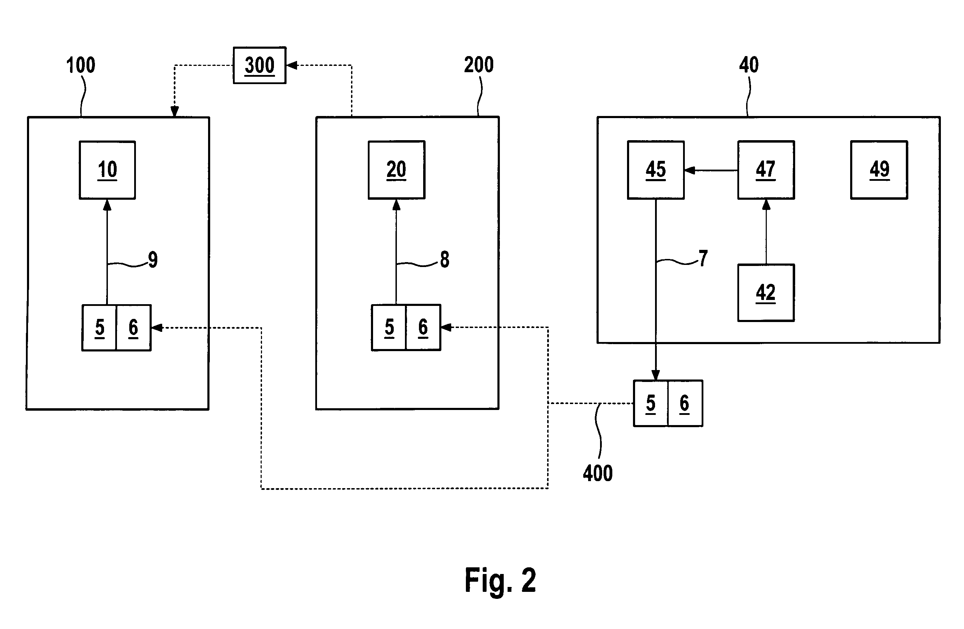

Es wird erneut auf Fig. 2 sowie auf das dazu beschriebene Beispiel 2 Bezug genommen. Der Materialrücklauf (400) veranschaulicht hier schematisch, auf welchem Weg eine Rückgabe von Verpackungseinheiten, Verbrauchsmitteln und/oder Datenspeichern vom Endnutzer des analytischen Messegrätes an das werksferne System (200) oder an das werksnahe System (100) erfolgen kann. Insbesondere bei Rückgabe von Datenspeichern an das werksferne System (200) kann vorteilhaft die zuvor beschriebene Übertragungsvorrichtung (300) zur Übertragung von Daten (Messdaten, Messergebnisse, Systemdaten bzw. Systemzustandsdaten, etc.) vom werksfernen System (200) an das werksnahe System (100) genutzt werden.

### Beispiel 4

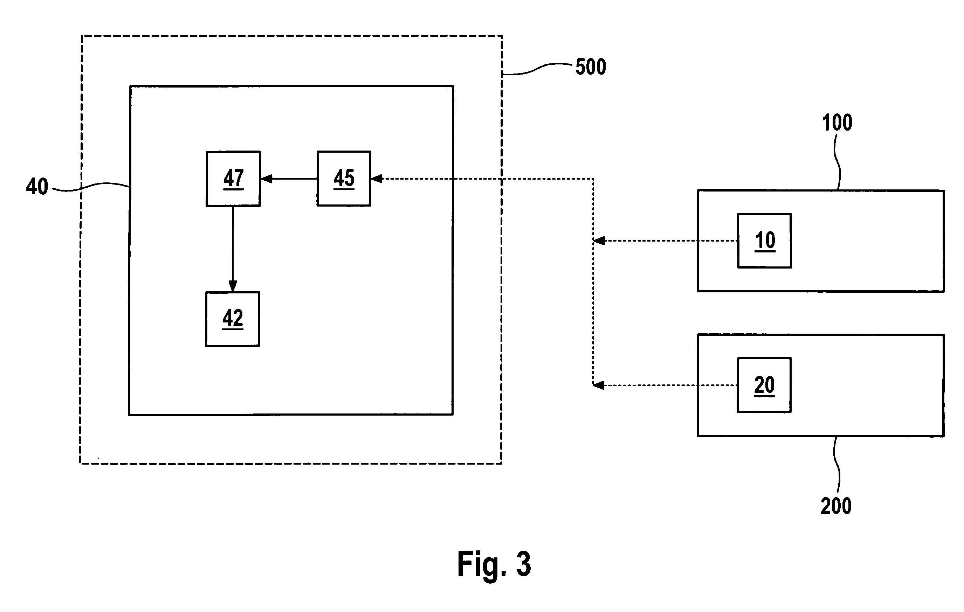

Es wird auf Fig. 3 Bezug genommen, um eine mögliche Datenübertragung zu veranschaulichen für den Fall, dass im Rahmen des werksnahen Systems (100) und/oder des werksfernen Systems (200) Daten auf ein bereits verpacktes analytisches Messgerät (40) übertragen werden sollen. Dies spielt insbesondere eine Rolle, wenn spezifische Einstellungen (Datum, Uhrzeit, Sprache, etc.) vorgenommen werden sollen. Die Verpackung (500) des analytischen Messgerätes (40) ist durch eine gestrichelt dargestellte Linie schematisch angedeutet. Hierbei ist die erste Datenempfangsvorrichtung (45) eingerichtet, um ohne eigene Spannungsquelle drahtlos Daten zu empfangen. Es ist somit möglich, Daten auf das analytische Messgerät (40) zu übertragen, ohne dass dessen Spannungsversorgung aktiv ist. So können auf diese Weise z.B. Daten ohne Einlegen einer Batterie oder eines Akkus auf das Messgerät übertragen werden. Vorliegend umfasst die erste Datenempfangsvorrichtung (45) ein Transponder-Lesemodul zum Auslesen von in einem Transponder mit RFID-Tag (wie zuvor beschrieben) gespeicherten Daten, wobei das Transponder-Lesemodul eingerichtet ist, um ohne eigene Spannungsquelle drahtlos Daten von einer externen Datenübertragungsvorrichtung (10) und/oder (20) zu empfangen.

## Patentansprüche

1. Analysesystem mit erweiterter Benutzerinformation, umfassend
- ein tragbares analytisches Messgerät zur Messung von Stofflconzentrationen in fluiden Medien, das eine erste Datenempfangsvorrichtung zum Auslesen von in einem beschreibbaren nichtflüchtigen Datenspeicher gespeicherten Daten und ein Anzeigeelement zum Anzeigen von Messergebnissen aufweist;
- eine Verpackungseinheit für Verbrauchsmittel, die zur Messung von Stoffkonzentrationen in fluiden Medien und/oder zur Vorbereitung einer solchen Messung verwendet werden, wobei der beschreibbare nichtflüchtige Datenspeicher der Verpackungseinheit zugeordnet ist;
- eine erste Datenübertragungsvorrichtung zum Übertragen von Steuerungsdaten auf den beschreibbaren nichtflüchtigen Datenspeicher; und
- eine zweite Datenübertragungsvorrichtung zum Übertragen von Anzeigedaten auf den beschreibbaren nichtflüchtigen Datenspeicher;
wobei das Anzeigeelement des tragbaren analytischen Messgerätes zum Anzeigen mindestens eines Teils der Anzeigedaten eingerichtet ist.

2. System nach Anspruch 1, **dadurch gekennzeichnet, dass** der beschreibbare nichtflüchtige Datenspeicher
- einen ersten Speicherbereich aufweist, der nur einmal mit Daten beschreibbar ist,
- einen zweiten Speicherbereich aufweist, der mindestens einmal und vorzugsweise mehrfach mit Daten beschreibbar ist, und
- gegebenenfalls einen dritten Speicherbereich aufweist, der ohne vorherige Authentifizierung mindestens einmal und vorzugsweise mehrfach mit Daten beschreibbar ist,
wobei der beschreibbare nichtflüchtige Datenspeicher gegebenenfalls ein Authentifizierungselement umfasst, durch welches ein Auslesen und/oder Beschreiben des zweiten Speicherbereichs erst nach erfolgreicher Authentifizierung autorisiert wird.

3. System nach einem der vorherigen Ansprüche, **dadurch gekennzeichnet, dass** der beschreibbare nichtflüchtige Datenspeicher drahtlos beschrieben und/oder ausgelesen werden kann und vorzugsweise einen Transponder, ein EEPROM-Speicherelement und/oder ein Flash-Speicherelement umfasst.

4. System nach einem der vorherigen Ansprüche, **dadurch gekennzeichnet, dass** das tragbare analytische Messgerät eine Analytkonzentration, insbesondere Glucose, in einer Körperflüssigkeit, insbesondere Blut oder interstitielle Flüssigkeit, misst.

5. System nach einem der vorherigen Ansprüche, **dadurch gekennzeichnet, dass** die Verbrauchsmittel ausgewählt sind unter Teststreifen zur Durchführung der Messung der Analytkonzentration, Sensoren zur Durchführung einzelner oder fortdauernder Messungen der Analytkonzentration in einem Körpergewebe, und Lanzetten zum Hervorbringen von Körperflüssigkeiten aus Körpergewebe.

6. System nach einem der vorherigen Ansprüche, **dadurch gekennzeichnet, dass** als Verpackungseinheit für die Verbrauchsmittel ein Behältnis zur magazinierten Aufbewahrung mehrerer der Verbrauchsmittel vorgesehen ist, wobei die Behältnisse insbesondere ausgewählt sind unter Trommel-Magazinen, Stangen-Magazinen, Reihen-Magazinen und Bandkassetten.

7. System nach einem der vorherigen Ansprüche, **dadurch gekennzeichnet, dass** eine dritte Datenübertragungsvorrichtung, die Bestandteil des analytischen Messgerätes ist, zum Übertragen von Messergebnissen und/oder von die Messung betreffenden Messdaten auf den beschreibbaren nichtflüchtigen Datenspeicher vorgesehen ist, wobei die dritte Datenübertragungsvorrichtung vorzugsweise eingerichtet ist, um die Messergebnisse und/oder Messdaten auf einen Speicherbereich des beschreibbaren nichtflüchtigen Datenspeichers zu übertragen, der ein Authentifizierungselement umfasst, durch welches ein Auslesen und/oder Beschreiben dieses Speicherbereichs erst nach erfolgreicher Authentifizierung autorisiert wird.

8. System nach einem der vorherigen Ansprüche, **dadurch gekennzeichnet, dass** die erste Datenübertragungsvorrichtung Bestandteil eines werksseitigen Systems ist, wobei das werksseitige System
- die Herstellung und Verpackung der Verbrauchsmittel in Verpackungseinheiten; und
- die Zuordnung des beschreibbaren nichtflüchtigen Datenspeichers zu den Verpackungseinheiten umfasst,
und wobei die zweite Datenübertragungsvorrichtung Bestandteil eines werksfernen Systems ist, wobei das werksferne System
- eine Vertriebsstelle zum Handel mit den Verpackungseinheiten, und/oder
- eine Distributionsstelle zur Distribution der Verpackungseinheiten an einen Endverbraucher umfasst.

9. System nach einem der vorherigen Ansprüche, **dadurch gekennzeichnet, dass** die Steuerungsdaten Reparaturdaten umfassen, welche die Korrektur fehlerhafter Funktionen des analytischen Messgerätes ermöglichen, und/oder Upgradedaten umfassen, welche eine Aktualisierung der Funktionen des analytischen Messgerätes ermöglichen, oder welche eine Außerbetriebnahme fehlerhafter Messgerätechargen oder nicht mehr genutzter Softwareversionen bewirken.

10. Analysesystem mit erweiterter Benutzerinformation, umfassend
- ein tragbares analytisches Messgerät zur Messung von Stoffkonzentrationen in fluiden Medien, das eine erste Datenempfangsvorrichtung zum Auslesen von in einem beschreibbaren nichtflüchtigen Datenspeicher gespeicherten Daten und ein Anzeigeelement zum Anzeigen von Messergebnissen aufweist;
wobei die erste Datenempfangsvorrichtung eingerichtet ist, um ohne eigene Spannungsquelle drahtlos Daten zu empfangen.

11. Analyseverfahren mit erweiterter Benutzerinformation, umfassend folgende Schritte:
(i) Bereitstellen von Verbrauchsmitteln, die von tragbaren analytischen Messgeräten zur Messung von Stoffkonzentrationen in fluiden Medien und/oder zur Vorbereitung einer solchen Messung verwendet werden, und von einer Verpackungseinheit für diese Verbrauchsmittel;
(ii) Übertragen von Steuerungsdaten auf einen beschreibbaren nichtflüchtigen Datenspeicher mittels einer ersten Datenübertragungsvorrichtung, und Zuordnung des beschreibbaren nichtflüchtigen Datenspeichers zu der Verpackungseinheit;
(iii) Übertragen von Anzeigedaten auf den beschreibbaren nichtflüchtigen Datenspeicher mittels einer zweiten Datenübertragungsvorrichtung;
(iv) Auslesen der Steuerungsdaten und der Anzeigedaten aus dem beschreibbaren nichtflüchtigen Datenspeicher mittels einer ersten Datenempfangsvorrichtung, die Bestandteil des tragbaren analytischen Messgerätes ist;
(v) Anzeigen zumindest eines Teils der Anzeigedaten auf einer Anzeigevorrichtung, die Bestandteil des tragbaren analytischen Messgerätes ist; und
(vi) Verwendung der Steuerungsdaten zur Durchführung der analytischen Funktionen des tragbaren analytischen Messgerätes.

12. Analyseverfahren nach Anspruch 11, **dadurch gekennzeichnet, dass** in Schritt (i) als Verpackungseinheit für die Verbrauchsmittel ein Behältnis zur magazinierten Aufbewahrung mehrerer der Verbrauchsmittel bereitgestellt wird, wobei die Behältnisse insbesondere ausgewählt sind unter Trommel-Magazinen, Stangen-Magazinen, Reihen-Magazinen und Bandkassetten.

13. Analyseverfahren nach einem der Ansprüche 11 bis 12, **dadurch gekennzeichnet, dass** Schritt (ii) in einem werksseitigen System, wie in Anspruch 8 definiert, erfolgt, und/oder dass Schritt (iii) in einer Vertriebsstelle und/oder Distributionsstelle eines werksfernen Systems, wie in Anspruch 8 definiert, erfolgt.

14. Analyseverfahren nach einem der Ansprüche 11 bis 13, **dadurch gekennzeichnet, dass** in Schritt (iii) die zweite Datenübertragungsvorrichtung die Anzeigedaten auf einen zweiten Speicherbereich des beschreibbaren nichtflüchtigen Datenspeichers, der mindestens einmal und vorzugsweise mehrfach mit Daten beschreibbar ist, überträgt, wobei die Übertragung der Anzeigedaten in Schritt (iii) vorzugsweise erst nach erfolgreicher Authentifizierung durch ein Authentifizierungselement des beschreibbaren nichtflüchtigen Datenspeichers, durch welches ein Auslesen und/oder Beschreiben des zweiten Speicherbereichs des beschreibbaren nichtflüchtigen Datenspeichers autorisiert wird, erfolgt.

15. Analyseverfahren nach einem der Ansprüche 11 bis 14, weiterhin umfassend folgende Schritte:
(vii) mindestens eine Messung einer Analytkonzentration in Blut durch das analytische Messgerät, wobei man mindestens ein Messergebnis und/oder die Messung betreffende Messdaten erhält;
(viii) mindestens teilweises Übertragen des Messergebnisses und/oder der Messdaten auf den beschreibbaren Datenspeicher mittels einer dritten Datenübertragungsvorrichtung, die Bestandteil des analytischen Messgerätes ist; und
(ix) Auslesen des Messergebnisses und/oder der Messdaten aus dem beschreibbaren Datenspeicher mittels
(ix.1) einer zweiten Datenempfangsvorrichtung, die Bestandteil eines werksfernen Systems, wie in Anspruch 8 definiert, ist; oder
(ix.2) einer dritten Datenempfangsvorrichtung, die Bestandteil eines werksseitigen Systems, wie in Anspruch 8 definiert, ist.

16. Analyseverfahren nach einem der Ansprüche 11 bis 15, **dadurch gekennzeichnet, dass** es mithilfe eines Systems nach einem der Ansprüche 1 bis 10 durchgeführt wird.
